# EUROPEAN PATENT SPECIFICATION

(11) **EP 2 756 070 B1**
(45) Date of publication and mention of the grant of the patent: **08.02.2017**
(21) Application number: 12805746.0
(22) Date of filing: 14.09.2012
(51) Int. Cl.: C12N 1/20, C12N 1/36, C12P 5/02, C12P 39/00, C02F 3/28, C02F 3/34, C02F 11/04, C02F 103/22

(54) **PRODUCTION OF BIOGAS FROM PROTEIN-RICH RESOURCES**
HERSTELLUNG VON BIOGAS AUS PROTEINREICHEN RESSOURCEN
PRODUCTION DE BIOGAZ À PARTIR DE RESSOURCES RICHES EN PROTÉINES

(30) Priority: 14.09.2011 HU P1100510
(43) Date of publication of application: 23.07.2014
(73) Proprietor: Szegedi Tudományegyetem, 6720 Szeged (HU)
(72) Inventor: KOVÁCS, Kornél, H-6723 Szeged (HU); KOVÁCS, Etelka, H-6727 Szeged (HU); MARÓTI, Gergely, H-6725 Szeged (HU); WIRTH, Roland, H-6300 Kalocsa (HU); ÁCS, Norbert, H-6723 Szeged (HU); RÁKHELY, Gábor, H-6726 Szeged (HU); BAGI, Zoltán, H-6726 Szeged (HU)
(74) Representative: Svingor, Adam
(86) International application number: PCT/HU2012/000092
(87) International publication number: WO 2013/038216

(56) References cited:
- WO-A1-2007/060683
- US-A1- 2008 282 733
- CHEN ET AL: "Inhibition of anaerobic digestion process: A review", BIORESOURCE TECHNOLOGY, ELSEVIER BV, GB, vol. 99, no. 10, 13 March 2008 (2008-03-13), pages 4044-4064, XP022526208, ISSN: 0960-8524, DOI: 10.1016/J.BIORTECH.2007.01.057 cited in the application
- ABOUELENIEN F ET AL: "Dry mesophilic fermentation of chicken manure for production of methane by repeated batch culture", JOURNAL OF BIOSCIENCE AND BIOENGINEERING, ELSEVIER, AMSTERDAM, NL, vol. 107, no. 3, 1 March 2009 (2009-03-01) , pages 293-295, XP026006831, ISSN: 1389-1723, DOI: 10.1016/J.JBIOSC.2008.10.009 [retrieved on 2009-03-06]
- BURAK DEMIREL ET AL: "The roles of acetotrophic and hydrogenotrophic methanogens during anaerobic conversion of biomass to methane: a review", RE/VIEWS IN ENVIRONMENTAL SCIENCE & BIO/TECHNOLOGY, KLUWER ACADEMIC PUBLISHERS, DO, vol. 7, no. 2, 31 January 2008 (2008-01-31), pages 173-190, XP019614339, ISSN: 1572-9826
- TALBOT ET AL: "Evaluation of molecular methods used for establishing the interactions and functions of microorganisms in anaerobic bioreactors", WATER RESEARCH, ELSEVIER, AMSTERDAM, NL, vol. 42, no. 3, 19 January 2008 (2008-01-19), pages 513-537, XP022427744, ISSN: 0043-1354, DOI: 10.1016/J.WATRES.2007.08.003

## Description

### FIELD OF THE INVENTION

The present invention relates to the field of waste processing and biogas production.

The invention relates to a process for production of microorganism consortium capable of producing methane containing biogas by fermenting a substrate containing at least 75%, preferably at least 90% protein, or highly preferably a protein monosubstrate through adaptation to the substrate, to a consortium containing bacteria obtainable by the process and/or proteolytic bacterium which belongs to the Gammaproteobacteria class; denitrifying bacterium; to cellulolytic, peptide and/or amino acid degrading and/or thiosulfate reducing bacterium that belongs to the Clostridia class, furthermore, to the use of the consortium for fermenting a substrate with high protein content with the production of methane-containing biogas and for enhancing biogas production.

### BACKGROUND OF THE INVENTION

In the 21st century the use of biomass plants and plant- based materials come to the foreground and even if they do not decrease the amount of CO₂ released to the atmosphere, they at least do not increase the amount of carbon-containing molecules in the circulation. Due to their numerous favourable properties renewable energy carriers show significant breakthrough. During the past years their costs have significantly decreased and at present, in certain applications they are competitive with fossil energy carriers. The anaerobic treatment of wastes, in addition to its environment protection role, can be regarded as a significant source of renewable energy.

In the nature the formation of gaseous methane, an energy carrier of biological origin, is created as a result of the synchronized work of several dozens microorganisms. Biogas is formed where in the nature organic materials are decomposed under oxygen-free conditions. A great number of microorganisms play a part in the decomposition process of organic materials of biological origin. Between members composing the system there is a complex system of interdependence, the so called syntrophic relationship.

At present in the European Union approximately there are 4-5000 biogas plants in operation and none of them utilizes starting materials rich in protein. At the same time huge amounts of protein-rich byproducts and wastes are generated in food production and processing, which are dangerous environmental pollutants and their disposal is an expensive and time-consuming task.

Every year tens of millions of tons of protein-rich hazardous material is generated continuously worldwide. By utilizing anaerobe treatment, in principle, from these materials renewable energy, biogas can be produced and the disposed fermentation residue can be used in agriculture as an excellent fertilizer substituent for soil reinforcement.

The approximately 400 meat works of the Hungarian meat processing sector, engaged in slaughtering and meat processing, produce about 300 thousand tons of pork, an amount obtained as a result of slaughtering 4-4.5 million pigs (www.ohki.hu). In meat and poultry industry waste recovery technologies must comply with several environmental protection requirements of the European Union:
- it has to be a low-waste technology
- principle of energy efficiency
- waste recovery
- strict regulations on the treatment of hazardous wastes.

In Hungary the average amount of wastes of animal origin is about 0.3 - 0.4 Mt/year.

Due to their pathogenic bacteria content, all wastes produced by the meat processing industry are considered as hazardous. Consequently, the disposal of these materials poses a considerable financial burden to the companies running the slaughter houses. It raises further difficulty that cleaning of meat industry waste waters is cumbersome, therefore, mechanical, biological as well as chemical purification should also be applied.

The production of broiler chickens gives rise to a similar problem all around the world.

While it is known that we could expect high biogas yields from the chemical energy stored in proteins, according to a doctrine widely spread in the professional literature concerning biogas, the ammonia originating from the decomposition of basic materials rich in proteins inhibits the microbiology of biogas production. This statemnt is supported by a number of experimental results. Therefore, with reference to the relevant scientific literature, the utilization of basic materials rich in protein is not recommended in biogas plants. The economical and effecient disposal and utilization of food industrial byproducts rich in protein is yet to be resolved.

Documents according to the relevant background art reflect this situation and indicate that the issue of biogas producable during the disposal of protein-rich wastes is a problem continuously studied, however, no solution has been provided.

The patent US6464875 *(Food, animal, vegetable and food preparation byproduct treatment apparatus and process)* relates to a system and method, which converts food industrial, plantal and animal byproducts to biogas and fertilizer. According to the description, the system is putatively capable of the anaerobe digestion of protein containing byproducts.

The patent US5185079 *(Anaerobic sequencing batch reactor)* relates to a new fermentor construction, which basically retains the active microbe mass performing biogas production in the batch fermentor by sedimenting it prior to discharge. In example 1 non-fat milk powder is utilized as substrate, but efforts have been taken to dilute it as much as possible. Furthermore, only a smaller portion of the dry material content of the fat-free milk powder is protein and the larger portion (about 50-60%) is lactose, as a result it cannot be considered to be a substrate with high protein content. The patent does not deal with the biotechnological aspects of biogas fermentation and the composition of the microbe community has not been studied or controlled either.

The GB2167639 *(Animal food from protein-containing waste materials), a document from the United Kingdom which* relates to a method in which slaughter house and food industry byproducts are treated with proteolytic enzymes. Biogas production was not emphasized. In this case the end-product is a suspension suitable for feeding animals.

This method can be really suitable for the disposal of byproducts, however, the cost of proteolytic enzymes is rather high and the suspension with high protein content in itself is not satisfactory for animal feeding. Thus, if such suspension is produced in high quantities, the storage and transportation of this large amount will impose additional expenses on animal breeders.

The JP2005161173 *(Method and apparatus for treating protein-containing wastewater by methane fermentation)* also discloses a method and equipment which are useful for treating protein- containing waste waters; the protein- containing water is subjected to lactic acid fermentation, as a result of which proteins become the separated and coagulated in the water, and only after this does methane fermentation take place. According to the description, waste water is separated in different fermentation steps and its different fractions are probably treated in various manners in further steps. In the disclosure there is no reference to the highest protein level of waste waters treatable by this procedure. A further disadvantage is that, in order to implement the solution, a separate factory, plant or facility should be built, as a consequence it demands considerable financial investment.

The US2008000106836 *(A system and method for thermophilic anaerobic digester process)* publication relates to a method for the biochemical treatment of waste waters, which is suitable for processing wastes generated in the milk and meat processing industry. The anaerobe treatment of the waste waters takes place in a two-chambered bioreactor, therefore, it has significant equipment requirements. The waste water is blended with mixed waste of animal origin in 3-5% as well as with dispersed waste.

The patent application MD2004000000005 *(Process sewage water biochemical treatment)* discloses a system that comprises a thermophyl anaerobe fermentation system as well as different independent modular units for promoting the production of biomethane from various organic wastes, e.g. communal organic waste, byproducts from restaurants and breweries. The disclosure mentions a stirring tank for the pre-treatment of wastes, in which the produced ammonia toxic to the biogas process transforms into ammonium ions. It is well-known that it is a pH-dependent process, therefore, it does not require a biotechnological action, however, for the purpose of the disposal of ammonia a separate technological procedure is required to be performed in a separate reactor.

The Hungarian patent application HU199500021401 *(Method for recycling partially decomposed protein waste of fibrous and woven composition and other hazardous waste by thermal disintegration and fementation)* discloses a method where first the wastes are subjected to thermal decomposition, then anaerobe fermentation process follows. In the disclosure a commonly-known inoculum is used, i.e. the microbe population is not adapted to the protein waste and it is not generated accordingly.

Solutions disclosed in HU200402444*,* HU195978 and HU224974 (Microbiological method for decomposing keratin...) intensifies the biotechnological processes by adding a single suitable bacterium strain to the microbe community. Decomposition of substrate with high protein content (animal hair) is only mentioned in HU224974*,* however, this patent does not disclose either the adaptation of a microorganism consortium to the decomposition of such substrate with protein monosubstrate or the utilization of such consortium for decomposition of such substrates. In order to that the only strain belonging to the Bacillus genus and containing keratinase enzyme should be able to decompose animal hair, it should be autoclaved at 135°C for at least 20 minutes.

The method disclosed in JP20050070796 *(Methanation method and apparatus)* comprises a methane fermentation treatment of organic wastes containing oils and fats, and an anaerobe acidic fermentation treatment for protein-rich wastes.

The first step of the process set forth in DE19991046299 *(Fermentation of carbohydrate, fat and protein-containing biological waste, cellulose-containing waste, sewage sludge, paper sludge and whey)* is the simultaneous fermentation, agitation and the homogenization of mixed composition wastes. Then the mixture is heated to 70°C and after 1 hour it is cooled back to 30-33°C. An enzyme solution performing aerobe hydrolysis is added to not readily degradable wastes, then after 3 days the material is subjected to anaerobe decomposition. After a further 3 days 10% paper sludge and 5% whey are added to the system.

The DD282673 *(Verfahren zur Gewinnung von Biogas aus schwer metabolisierbaren Abwasserschlämmen*...*")* discloses a method for the production of biogas from waste water of microbiological protein production proceeding from mineral oil distillate. However, the waste water comprises mainly mineral oil derivatives and it does not contain proteins.

The method set forth in SE19940001265 *(Production of combustible gases especially methane)* encompasses the heating of basic waste materials originating from slaughter house byproducts in order to initiate the growth of thermophyl bacteria and to remove pathogens from the system. Susequently the temperature is decreased to initiate mesophil biogas production.

WO2007/060683 *(A microbial consortium and use thereof for liquefaction of solid organic matter)* discloses a microbial consortium for the liquefaction of solid organic matter, comprising bacteria from several genera. The solid organic matter may be generated from tanning industries, poultry farms, food processing industries, etc. This application also describes a method for the liquefaction of the solid organic matter, which comprises treating the solid organic matter with 100 % to 300% w/v, of water containing fermentative microorganisms with a minimum population density of 10² per ml. Further treatment conditions are anaerobic conditions, a pH of not less than 3.0 and a treatment period of minimum 5 days.

The above patent disclosures and patents do not reveal anything about the composition, effects and applicability of the microbiological system. None of the above descriptions describes the production of microorganism consortium adapted to a clean protein substrate (protein monosubstrate). The problem of ammonia production - if it is dealt with at all - e.g. is resolved by a disposal taking place in a separate step.

Professional literature also studies the above outlined problem area, partly from the practical and partly from the theoretical point of view.

In the study of Ruiz, I. et al. [Bioresource Technol., 60(3):251-258, 1997.] the protein content of the treated slaughter house waste was 70% and it contained mainly blood. The authors measured the amount of methane produced and they found that by using anaerobe filter less methane was produced than in the UASB reactor. They concluded that anaerobe treatment systems can be applied well for the disposal of slaughter house wastes as well as for the production of renewable energy.

Wu, G. et al., [Bioresource Technol., 100(19):4326-4331, 2009.] studied the possibilities of the anaerobe fermentation of meat and bone meal in samples with different MBM solid contents. The highest MBM solid content was 10%. According to the publication, under the conditions applied the generated ammonia only reversibly inhibited the methane production. The system was capable of adapting to a high ammonia concentration (998 mg/l).

Cuetos, M.J. et al., [Biochem. Engn. J., 40(1):99-106, 2008.] studied the anaerobe disposal of slaughter house wastes with the co-decompostion of it with communal waste. They found that the efficiency of fermentation and biogas production was satisfactory after the microbe population performing the fermentation and biogas production had been suitably adapted to the high fat and ammonia concentration.

Hejnfelt, A. and Angelidaki, I., [Biomass and Bioenergy, 33(8):1046-1054, 2009.] disclose the possibilities of mesophyl and thermophyl anaerobe decomposition of slaughter house wastes. The authors studied the possibilities of blood, raw wastes and bone meal disposal and their application in biogas production. They also studied the biogas production of the combination of slaughter house wastes and slurry and in the case of co-fermentation they found a significant increase in biogas production compared to the slurry.

Luste, S and Luostarinen, S., [Bioresource Technol., 101(8):2657-2664., 2010.] studied the anaerobe fermentability of various mixtures of meat industrial wastes and waste water sludge. Their principal conclusion was that co-fermentation improves decomposition and biogas production parameters.

In their review Chen, Y. et al., [Bioresource Technol., 99(10):4044-4064, 2008] analyzed the inhibition processes of anaerobe decomposition. The publication discusses several important factors, such as the problem of ammonia production and the importance of ammonia in inhibiting methane production in the case of protein-rich substrates. Although in their review they theoretically describe that stable fermentation can be reached with manure containing 5 g N/L ammonia, however they stated that methane production was lower compared with substrates containing less ammonia. The authors see the solution to the problem in co-fermentation performed with other substrates or in the binding (e.g. with zeolite or galuconite) or the removal of ammonia. The accurate and detailed review does not discuss the importance and the composition of the consortia.

According to the art, the inhibiting effect of ammonia generated in the process of anaerobe protein fermentation was commonly known. The possibility is mentioned in several publications that under certain conditions and up to a certain ammonia concentration biogas-producing microbe communities can be made to become adapted to an amount of ammonia severalfold of the tolerance threshhold. These methods generally attempt to provide a solution to the problem by the co-fermentation with some other organic wastes, which results in the shift of the carbon/nitrogen ratio to a favourable direction. Most of the publications concluded that proteins can only be decomposed and converted to biogas really effectively in the presence of a co-substrate (co-fermentation). Even in this case fermentation and sufficient biogas production can be maintained with good results only along with the sustainment of a relatively low or limited protein content. No publication was found in the state of the art about the microbial composition of the system or its effects and usability. No technology is known that is useful for the effecient and persistent anaerobe decomposition of protein-containing monosubstrate together with continuous biogas production.

However, due to its energy content, the biomass rich in proteins may constitute an excellent basic material provided the anaerobe degradation of protein-based organic material can be resolved by the elimination of the disadvantages of the art. The present invention provides a possible solution to this issue.

The present invention is based on the discovery that it is possible to make a microorganism consortium already accustomed to a conventional biomass to adapt to a substrate with high protein content, even to a protein monosubstrate without using a co-substrate and this way the problem of ammonia production can also be solved and it becomes unnecessary to dispose of the ammonia through an other way.

With the consortium adapted to the protein substrate, utilization of protein-rich organic compounds and by-products of agricultural and processing industrial origin for biogas production becomes possible.

### THE SUMMARY OF THE INVENTION

In the present invention a new method was developed, which is suitable for creating a microbiological community capable of adapting to protein-rich substrates and producing a stable and good biogas production in the long run. The microbe community (microorganism consortium) of the invention adapted to a special basic material is capable of producing by 30-50% more biogas from a given amount of protein- rich basic materials than non-adapted systems do.

The consortium created by the method can be applied in both batch and continuous biogas producing systems, accordingly it also can be utilized in already operating systems, and there is no need for building special new or auxiliary reactors; the method can be utilized in biogas producing equipment already in operation.

Microbiological taxonomy groups in the consortium which play an important role in the decomposition of protein-rich basic materials were determined and characterized metagenomically.

Based on the results, there is possibility to create a microorganism consortium which is or can be taxonomically characterized and has advantageous composition and characteristics, wherein said consortium can also be used as an inoculation material. On the basis of the results, operators of biogas plants utilizing basic materials rich in protein can be advised to provide stable operational conditions and a microbiological product can be produced which provides solution to the problem at the operational level of the plant.

### Adaptation processes

Thus, the invention provides a method for the production of microorganism consortium which is capable of fermenting a protein-containing substrate containing at least 75% of protein, preferably at least 80%, 85%, 90%, or 95% of protein, highly preferably protein monosubstrate and an organic dry-matter content of at least 4 g/l, preferably 5 g/l, more preferably at least 6 g/l relative to the fermentor volume, while producing methane-containing biogas by the adaptation to the substrate, wherein the method comprises the following steps:
- providing biogas-producing microorganism consortium cultured in anaerobe conditions until complete fermentation, preferably on manure or on plantal substrate or the mixture thereof,
- the microorganism consortium and a protein monosubstrate are added to the biogas fermentor,
- the microorganism consortium is cultured on the protein- monosubstrate thereby giving rise to the fermentation of the protein containing substrate,
- protein monosubstrate is added several times and in further portions (aliquots or doses) to the fermentation mixture (broth) to the biogas fermentor during the culture period,
- the fermentation process taking place in the fermentation mixture is monitored,
- on the basis of the monitoring it is determined that the microorganism consortium is stable i.e. when at least during the fermentation the concentration of one or more measured organic acids remains essentially unchanged, i.e. the change in concentration of the measured organic acids is not more 30%, and the change in biogas yield is not more than 30% for at least approximately 3 weeks, and it is adapted to the substrate provided that, according to the result of monitoring, the consortium is stable and produces biogas having preferably at least 50%, more preferably 60%, most preferably 70% methane on the substrate,
- stable and substrate-adapted microorganism consortium is collected from the fermentation mixture,
wherein the biogas producing microorganism consortium comprises at least proteolytic and/or peptidolytic, acetogenic and methanogenic microorganisms.

Preferably, in the adaptation method of the invention the protein monosubstrate
- is added at specific times, preferably regularly, at least for (i.e. during a period of) 20 days, preferably for at least 30 days, 40 days, 50 days, 60 days or highly preferably for at least 70 days or 80 days to the fermentation mixture, and/or
- in a first period it is added in equal portions, then in a second period in increasing amounts of portions to the fermentation mixture.

Preferably, the protein-monosubstrate is added to the fermentation mixture at least daily, at least every 2nd day, at least every 3rd day, or at least every 4th day, and every 14th day or 10th day at the most or every 7th day at the most. Highly preferably, the addition is performed once or more times weekly.

Preferably, at the time of monitoring the fermentation process one or more steps of the following steps are performed at least on two occasions:
- data is collected about the composition of the microorganism consortium in the fermentation broth,
- the concentration of one or several volatile organic acids (preferably acetic acid and/or propionic acid) is determined in the biogas reactor, preferably 1 or 2 days prior to adding the substrate portion,
- the protease activity in the fermentation mixture is determined, preferably at most 120 minutes, preferably at most 90 minutes, highly preferably at most 60 minutes, 40 minutes or 30 minutes after the addition of the substrate portion,
- the amount of biogas produced is measured in the biogas reactor.

According to a preferred variation of the monitoring process
- data is obtained - preferably by using metagenomic method - about the composition of the microorganism consortium in the fermentation mixture at least twice, at least with a difference of at least 2, 3, 4, or 5 weeks and if compared with the first data extraction at least on the second occasion
- it comprises bacterium species belonging to the Gammaproteobacteria class, preferably to the Pseudomonadales order in a significantly increased ratio, preferably in at least 20%, more preferably in 20-50%, preferably in 22-40%, and
- it comprises bacterium species belonging to the Clostridia class, preferably to the Clostridiales order and to the Thermoanaerobacterales order in a significantly decreased ratio, preferably in a ratio of less than 30%, more preferably in 10-40%, highly preferably in 20-30%
then the data is considered to indicate adaptation.

Preferably, the inventive method of producing the consortium is performed anaerobically or near anaerobically.

Preferably, in the method of the invention the protein monosubstrate is added to the fermentation mixture as solid material or in a solution of at least 30%, at least 50%, at least 70% or at least 90% concentration.

At least one type of microorganism may be isolated from the adapted microorganism consortium. Optionally, at least one isolated strain of microorganism is obtained or cultured. Optionally, the microorganism or strain of the microorganism is tested for its biogas production ability or further selected on the protein substrate. Optionally, the method of the invention is performed with the microorganism or the strain of microorganism. Optionally, the microorganism or the strain of microorganism is used in itself or admixtured to the consortium of the invention.

Preferably, the adapted microorganism consortium comprises the following microorganisms:
among species belonging to the Clostridiales order
   - a species with cellulolytic activity,
   - a species capable of reducing metal ions (preferably iron, cobalt, manganese and selenium ions) and/or among species belonging to the Pseudomonadales order
   - species with proteolytic activity.

In the monitoring process at least one or preferably both of the following steps are performed highly preferably at least 3, 4, 5, 6, 7 or at least 8 times:
- the concentration of one or several volatile organic acids is determined in the biogas reactor,
- the protease activity is determined in the fermentation mixture.
Expediently, the concentration of one or several volatile organic acid is maintained under a given level by adjusting, preferably limiting the substrate addition, thus providing the adaptation of the microorganism consortium and preventing the system from perishing or breaking down. Highly preferably, the acetic acid concentration is less than 5 g/l, more preferably less than 3,5 g/l and highly preferably it is between 1-2.5 g/l.

Expediently, by adjusting, preferably limiting the substrate addition, an increase, preferably a consistent elevation is reached in the protease activity.

Highly preferably, the amount of biogas produced is measured in relation to or compared with the protein content of the added substrate in the biogas reactor, and provided that, in the event of an increase in the amount of the added substrate, the amount of the produced biogas increases, this is considered as a data indicative of adaptation; and optionally when the rate of increase in biogas production decreases in relation to or compared with the increase of the amount of substrate added, the microorganism consortium is considered stable and adapted to the substrate. Highly preferably, where the initial protease activity is 100%, the protease activity measured in stable condition is preferably 200%, more preferably 300%, highly preferably 350%.

### Microorganism consortia

The invention provides a microorganism consortium capable of producing biogas, wherein the microorganism consortium comprises proteolytic and/or peptidolytic, acetogenic and methanogenic microorganisms, and which can be obtained according to any of the adaptation or habituation methods of the invention, and wherein the biogas-producing microorganism consortium is capable of fermenting a substrate comprising at least 90% protein and at least 4 g/l, preferably at least 5 g/l, more preferably at least 6 g/l organic dry material calculated based on reactor volume, with simultaneous production of methane-containing biogas, and the microorganism consortium comprises at least
- a proteolytic bacterium belonging to the Gammaproteobacteria class,
- a bacterium having denitrifying activity,
- a cellulolytic, peptide degrading and/or amino acid degrading and/or thiosulfate reducing bacterium of the Clostridia class, and preferably
- in a ratio of at least 20%, more preferably 20-50%, preferably 22-40% bacterium species belonging to the Gammaproteobacteria class, preferably to the Pseudomonadales order, and
- in a ratio of less than 50%, more preferably 10-40%, highly preferably 20-30% bacterium species belonging to the Clostridia class, preferably to the Clostridiales order and to the Thermoanaerobacterales order
wherein the biogas producing microorganism consortium is capable of fermenting a protein monosubstrate and preferably is capable of producing biogas of at least 500 ml/g, preferably at least 600 ml/g, highly preferably at least 700 ml/g in relation to organic dry-matter content, and is capable of maintaining the production of biogas for at least 1 month or more preferably for at least 2 months.

Furthermore, the microorganism consortium of the invention preferably comprises
- a metal ion reducing bacterium of the Clostridia class, and/or
- a methanogen bacterium of the Clostridia class, and/or
- an amino acid and/or sugar metabolizing and/or thiosulfate reducing bacterium of the Synergistia class, and/or
- a proteolytic and/or nitrate fermenting and/or nitrite fermenting bacterium of the Bacilli class, and/or
- a methanogenic microorganism belonging to the Archea domain.

According to a preferred embodiment of the microorganism consortium of the invention
- the proteolytic bacterium of the Gammaproteobacteria class belongs to the Pseudomonadales order, preferably selected at least form Pseudomonas aeruginosa, Pseudomonas entomophila, Pseudomonas fluorescens or Pseudomonas putida species,
- the denitrifying bacterium belongs to the Gammaproteobacteria class and/or to the Synergistia class, preferably it is a bacterium of the Pseudomonadales order and/or Synergistales order, highly preferably it is at least Pseudomonas stutzeri, Azobacter vinelandii or other bacterium that belongs to any of the above orders,
- the cellulolytic, peptide-metabolizing and/or amino acid metabolizing bacterium belongs to the Clostridia class, preferably belongs to the Clostridiales order, preferably it is selected at least from Clostridium celloliticum, Clostridium sticklandii, Clostridium perfingens, Alkaliphilus peptidofermentans and Desulfitobacterium hafniense.

According to further preferred embodiments of the invention, in the microorganism consortium
- the metal ion reducing bacterium of the Clostridia class is preferably selected from the Alkaliphilus metalliredigens and Desufotomaculum reducens, and/or
- the hydrogen-producing and polymer-degrading bacterium of the Clostridia class belongs to the Thermoanaerobacterales order and is preferably Caldicellulosiruptor saccharoliticus, and/or
- the amino acid and/or sugar-metabolizing and/or thiosulfate- reducing bacterium of the Synergistia class belongs to the Synergistales order, preferably, it is selected from Aminobacter colombiense, Anaerobaculum hydrogeniformans, Termoanaerovibrio acidaminovorans and Dethiosulfovibrio peptidovorans, and/or
- the proteolytic and/or nitrate fermenting and/or nitrite-fermenting bacterium of the Bacilli class belongs to the Bacillales order and preferably it is selected from at least Bacilus cereus, Bacillus amyloliquefaciens and Bacillus subtilis, and/or
- the methanogenic microorganism of the Archea domain is selected from microorganisms belonging to the Methanosarcina, Methanothermobacter, Methanococcoides, Methanoculleus and Methanococcus order, preferably it is selected from Methanosarcina barkeri, Methanothermobacter thermoautotrophicus, Methanococcoides burtonii, Methanoculleus marisnigri and Methanococcus voltae, highly preferably a Methanothermobacter species living in syntrophy with Termoanaerovibrio acidaminovorans, and it is preferably Methanothermobacter thermoautotrophicus.

The microorganism consortium of a highly preferred embodiment comprises at least three microorganisms selected from the following:
- Alkaliphilus peptidofermentans
- Aminobacterium colombiense
- Desulfitobacterium hafniense
- Dethiosulfovibrio peptidovorans
- Pseudomonas fluorescens.

Optionally, the microorganism consortium of the invention comprises a microorganism strain isolated or obtained from the microorganism consortium of the invention and optionally it is enriched in one of its components.

According to a highly preferred embodiment, the microorganism consortium of the invention is capable of fermenting protein substrates. Preferably, the microorganism consortium of the invention is capable of producing, relative to dry organic matter, an amount (volume) of at least 500 ml/g, preferably at least 600 ml/g, highly preferably at least 700 ml/g, highly preferably at least 900 ml/g biogas on protein monosubstrate. Highly preferably, the microorganism consortium of the invention is capable of fermenting blood substrate and, relative to the dry organic matter obtained, it can produce an amount of at least 500 ml/g, preferably at least 600 ml/g, highly preferably at least 700 ml/g or at least 800 ml/g or at least 900 ml/g, highly preferably at least 1000 ml/g biogas. Highly preferably, the microorganism consortium of the invention is capable of fermenting casein substrate and, relative to the dry organic material, it can produce an amount of at least 1000 ml/g, preferably at least 1300 ml/g, highly preferably at least 1600 ml/g of biogas on casein.

### Uses of the microorganism consortium

Furthermore, the invention provides the use of the microorganism consortium of the invention for fermenting protein-rich substrate along with the production of methane-containing biogas. Preferably, the protein-rich substrate contains protein or protein-monosubstrate of at least 75%, preferably at least 80%, 85%, 90%, highly preferably at least 95%.

Furthermore, the invention provides the use of the microorganism consortium of the invention for improving biogas production by adding the microorganism consortium to a biogas fermentor comprising a protein-containing substrate; additionally, the use of the microorganism consortium of the invention for improving biogas production by adding a protein-containing substrate to a biogas fermentor comprising the microorganism consortium.

### Methods for fermenting protein-rich substrate with biogas production

The invention provides a method for fermenting protein-rich substrate with biogas production, wherein
- a fermentation mixture comprising substrate of at least 70% protein content and the microorganism consortium defined above or in the description is provided in a biogas fermentor,
- the fermentation mixture is fermented,
- the generated biogas is collected or utilized.

Preferably, a continuous reactor is applied and the substrate is added to the system continuously or in portions. According to a further embodiment of the invention, a batch reactor is used and the substrate is added in portions.

According to a preferred embodiment, during fermentation, given portions of the protein-containing substrate, preferably pre-determined ones or portions used regularly are added to the fermentation mixture in the biogas fermentor during the culture period.

Preferably, the protein-containing substrate is a protein-rich substrate or it comprises at least 70%, preferably at least 80%, 85%, 90%, highly preferably at least 95% of protein or it is a protein-monosubstrate.

Preferably, the fermentation is maintained for at least 1 month, more preferably at least for 2 months, highly preferably at least for 6 or 12 months. Preferably, the protein-containing substrate is added to the fermentation mixture at least daily, at least every 2 days, at least every 3 days, or at least every 4 days, and every 14 days at the most, every 10 days at the most or every 7 days at the most. Highly preferably, addition is performed on one or more occasion per week.

Preferably, the amount of portions added is determined from the portion necessary for reaching the maximum biogas yield during the adaptation, and preferably, at least 50%, preferably, at least 70% or 90% of said portion, preferably, at most 200% or at most160% or at most 120% of the portion is used.

According to a preferable variation, the substrate with high protein content, preferably protein monosubstrate is used in at least 1 g/l, 2 g/l, 5 g/l, 10 g/l or 15 g/l, and/or at most 100 g/l, 50 g/l, 30 g/l or 20 g/l relative to fermentor volumes.

Preferably, biogas is produced in the process of fermentation and preferably, the biogas is collected and/or utilized. In an embodiment a part of the biogas is utilized, e.g. continuously utilized, while the unused (excess) biogas is collected and stored. Highly preferably, the biogas is collected and/or utilized continuously during the fermentation.

Preferably, the system is monitored, preferably, by the application of any of the methods described above.

According to an embodiment of the invention, fermentation and biogas production are accomplished as the continuation of the adaptation process.

Furthermore, a method is disclosed herein for characterizing a system capable of biogas production by substrate fermentation of a microorganism community, preferably, the applicability of the method for processing a substrate of high protein content, in the process of which the system is characterized by the composition of the microorganism community, preferably by metagenomic methods. As a consequence of the above procedure, the operational safety and the efficiency of the biogas-producing system can be improved.

Preferably, the use and/or method of the invention is performed anaerobically or near-anaerobically.

Preferably, the protein-containing substrate is blood, e.g. blood waste, preferably blood of mammalian origin, such as swine blood, cattle blood, sheep blood, goat blood; blood of bird origin, highly preferably poultry blood, such as chicken blood, duck blood, turkey blood, goose blood; blood of fish origin, such as the blood of sea fish, fresh water fish, said fishes collected by net fishing or fish harvesting.

### DEFINITIONS

As used herein, the term "microorganism consortium" is a community of several microorganism species, where the metabolic product of at least one microorganism species is used by at least another microorganism species as nutrient during culturing of the microorganism consortium.

The term "culturing microorganism consortium" has the meaning that the consortium is maintained for defined and/or measured period in such a defined and/or measured conditions where the individuals of at least two species grow or able to grow in the microorganism consortium.

A microorganism is "stable", when at least during the fermentation the concentration of one ore more measured organic acid remains essentially unchanged, e.g. the change in concentration of the measured organic acids is not more than 40%, preferably not more than 30% or 30%, highly preferably not more than 15% or 10%, for at least approximately for 2 weeks, preferably for at least approximately for 3, 4 or 5 weeks, more preferably for at least approximately for 6, 7, 8 or 9 weeks. Preferably the biogas yield is constant, e.g. the change in biogas yield is not more than 40%, preferably not more than 30% or 30%, highly preferably not more than 15% or 10%.

As used herein, the term "substrate" refers to a material suitable to being a nutrient for at least one species in the microorganism consortium.

The term "protein-containing substrate" is a substrate which contains protein.

The term "substrate with high protein content" is a substrate which contains more than 70%, preferably at least 75%, 80%, 85%, 90% or 95% protein.

The term "protein monosubstrate" is a substrate with high protein content which basically contains protein, preferably contains at least 95%, 97%, 98%, 90% or 99% protein.

The substrate with high protein content is preferably protein monosubstrate, preferably blood or casein.

As used herein where the protein content is given in %, the % is meant weight%.

In a list, the "at least" n, m, o, etc. value is meant that the "at least" term applies to the m, o, etc. values as well.

The term "biogas" is an aeriform material which is produced during the cultivation of microorganism consortium on a substrate.

The term "methane containing biogas" is a biogas which contains at least 20%, 30%, 40%, highly preferably at least 45%, 50%, 55%, 60% or 65% methane. Characteristically the biogas contains carbon dioxide, e.g. 20-40% carbon dioxide, and other aeriform materials, such as hydrogen, hydrogen sulfide, ammonia, NOx, CO, N2, in a smaller amount.

The term "fermenting or fermentation" meant a process where during the cultivation of a microorganism consortium on a substrate, due to the metabolism of the microorganisms the substrate transforms chemically and biogas is produced.

The "biogas fermentor" is a vessel, preferably a reactor, where the fermentating or fermentation takes place along with biogas production or the vessel is suitable for this purpose.

The term "fermentation mixture" is a material of the microorganism consortium and the substrate or a material comprising these.

The term "anaerobe conditions" refers to conditions lacking oxygen, i.e. where the concentration or partial pressure of the oxygen is lower than that of the environment. Preferably, the anaerobe conditions refer to conditions lacking the oxygen, where the obligate anaerobe microorganisms are capable of growing or can be cultured.

The term "complete fermentation of the substrate" means that after culturing a microorganism consortium on the substrate - preferably under anaerobe conditions - the substrate is not replaced, thus the biogas production is left to decrease, which indicates the progress of the process; the cease of the biogas production indicates the end of the complete fermentation.

The term "monitoring the fermentation process" means that in the process of fermentation data is collected several times in the biogas reactor by measuring the physical or chemical properties of the fermentation mixture or the biogas. Frequently the monitoring is carried out by collecting samples and performing measurements on the sample. Thus, e.g. the pressure, temperature, volume of the biogas, or the ratio, concentration, partial pressure, etc. of any of biogas components (e.g., methane, carbon dioxide, hydrogen, hydrogen sulfide) can be measured.

Furthermore, the temperature, density, etc. of the fermentation mixture also can be measured. Preferably, the composition of the fermentation mixture, the ratio or concentration of any components (e.g., volatile fatty acids, ammonia, protein content, peptide content, sugar content, etc.) of the fermentation mixture can be measured, or the taxonomical classification, germ number, weight, composition, etc. of the microorganisms can be determined. The determination of the ratio and taxonomical classification of the microorganisms is preferably carried out by using metagenomic methods.

Action performed or carried out "at specific intervals", e.g. actions performed at determined or measured time intervals during the measurements or sample collection.

Action performed or carried out "on regular basis" or "regularly", e.g. during a measurement or sampling, means an action performed or carried out at specific intervals, where a mathematical correlation - e.g., constant, or directly proportional with the number of actions, or possibly gradually changing correlation - can be found between time intervals.

### SHORT DESCRIPTION OF THE DRAWINGS

Figure 1: Change in the amount of biogas produced from per unit organic material in the biogas production system adapted to the protein substrate.
Figure 2: Accumulation of organic acids as a function of the amount of protein added.
Figure 3: Change in protease activity as a function of the amount of casein added to the system.
Figure 4: Biogas production of protein adapted and not adapted microbe population in relation to per unit organic material.
Figure 5: Time line changes of continuous biogas fermentation with the addition of protein monosubstrate; in the system the microbe population capable of biogas production from the protein becomes gradually enriched and accordingly the daily gas yields also increases.
Figure 6: A protein adaptation and load test in a continuous fermentor.
Figure 7: The increase in protease activity for the addition of casein substrate.
Figure 8A: Protein adaptation and load test in a continuous fermentor.
Figure 8B: The increase in protease activity for the addition of blood protein.
Figure 9: The mean daily gas yield as a function of the substrate quantity during fermentation and biogas production performed on protein substrate.
Figure 10: The distribution of the initial microbe population according to classes in the Bacteria domain.
Figure 11: The 3 most abundant methanogene of the Archea domain in the different type of fermentors.
Figure 12: Family distribution in the Bacteria domain found in the blood containing fermentor at the initiation and at 5 weeks after initiation.
Figure 13: After adaptation the 3 most abundant methanogene of the Archea domain in the blood fermentor.
Figure 14: Family distribution in the Bacteria domain found in the swine blood containing fermentor at the initiation and at 5 weeks after initiation.
Figure 15: After adaptation the 3 most abundant methanogene of the Archea domain in the fermentor containing casein.

### DETAILED DESCRIPTION OF THE INVENTION

In nature the formation of gaseous methane as an energy carrier of biological origin occurs as a result of the synchronized work of several dozen microorganisms. Biogas is formed where in nature organic materials are decomposed under oxygen-free conditions. The process may be used for utilizing wastes through anaerobe treatment; thus in addition to protecting the cleanliness of the environment, renewable energy can also be produced.

Microorganisms decomposing organic materials of biological origin are classified into three main groups. The first group is responsible for the disintegration of polymers and microbes break down large molecular size organic molecules into simpler units. The group of acetogene bacteria utilizes the products created by the first group, which in turn generate organic acids and certain microbes also produce hydrogen. The third group is constituted by methanogenic microorganisms which produce the two main biogas components, methane and carbon dioxide. It is clearly visible that a complex system of interdependence, the so called syntrophic relationship, exists between the members constituting the system.

The type of the biotechnological process highly influences the methane content of the biogas. The nutrient composition of the basic material, the fermentation temperature and some other key parameters also influence the methane content; proteins and fats generate higher methane content.

In addition to methane, biogas contains 28-48% carbon dioxide and 1-2% other gases, such as hydrogen sulfide, carbon monoxide and ammonia. Hydrogen sulfide produced during the decomposition of proteins is a toxic gas which can cause corrosion of metal equipment.

By realizing the necessity of the disposal and the utilization of the huge amounts of protein-rich industrial and agricultural by-products in the form of biogas, is the present inventors developed a method which - by changing the composition of the microbe community performing the anaerobe decomposition - gradually adapts the biogas-producing system to protein basic materials exceeding several times the limit known in the professional literature. By developing the already known approaches, the inventors elaborated a method for the addition of protein-rich basic materials in increased amount and for the disposal of such byproducts in the form of protein monosubstrate (e.g., blood, casein) in biogas producing systems.

It was unexpectedly discovered that it is possible to develop a microbe population capable of decomposing substrate with high protein content in a biogas fermentor. On the basis of earlier experiments, protein is considered as a substrate inhibiting fermentation. This is based on the knowledge that protein contains nitrogen and sulfur in high quantities, from which ammonia, ammonium ion (from the nitrogen) and hydrogen sulfide (from the sulfur) are generated. Depending on the pH and the amount, these materials have an inhibiting effect on the fermentation process (according to earlier observations, when ammonia reaches a concentration exceeding 50 mg/l, it exerts a negative effect on methanogenes, while above the concentration of 1500-3000 mg/l, it causes a complete system block). The inventors have found that in the experiments where this statement was proved, protein was added to the system as a mixture with polysaccharides, which was more readily and quickly utilized by the microorganisms in the fermentor, thus protein became accumulated in the system and inhibited the fermentation process.

In laboratory experiments the microbe population of the biogas fermentor was successfully adapted to a high-level protein content and the fermentation process was maintained efficiently. In the experiments swine blood and casein served as protein substrates. According to the invention, any other substrate with high protein content, preferably protein monosubstrate can be applied, during the process of which the disadvantage of fermentation performed on the above mentioned co-substrate, i.e. the quicker fermentation of the substrate different from the protein does not occur.

Feasibly, during the process of adaptation the substrate with high protein content is added regularly or - in the case of a continuous reactor - continuously but in a controlled amount. Feasibly, the fermentation is started with a small amount of protein substrate, the amount of which is known to be tolerated by the given initial consortium and to the thus obtained fermentation mixture a protein substrate is added in an amount known to be tolerated for a given period at certain time intervals or during a given time unit.

It should be taken into account how much substrate should be added in a time unit to be able to avoid the break-down of the adapting system. Feasibly, this can be performed by monitoring the system.

In a preferred embodiment of the invention the system can be followed up by monitoring the concentration of the produced organic acids, such as volatile fatty acids, e.g. acetic acid and/or propionic acid. If the concentration of organic acids starts to increase or exceeds a limit determined empirically earlier, this is considered as data indicating the disruption of the balance of the adaptation or that of the production system.

Similarly, the performance capacity or efficiency of the fermentation mixture can also be monitored. A possibility to carry out this task is to measure the protease activity of the microorganism consortium in the mixture. In the process of adaptation the protease activity should gradually increase, while in the case of a stable system or a system that became stabilized, it should stagnate after the earlier increase. Similarly, the level of biogas production can be analyzed itself. In this case a principle, similar to that of the protease activity, applies.

For the purpose of metagenomic studies, samples were regularly (weekly) collected from the fermentors and subsequently analyzed. Naturally, the sample can be collected at any other prefixed time, reasonably, according to a predetermined schedule or regularly.

The samples were characterized.

In an embodiment of the invention, DNA was isolated from the initial and the week 5 samples so that, on the basis of the DNA sequence, species which constitute the microbe community living in the fermentors should be retrieved.

By metagenomic studies of microbe communities adapted in continuous fermenting conditions the inventors identified microbe groups and the most important species which change or accumulate in the biogas-producing system. By using a metagenomic approach, the changes occurring in the microbiological community adapted to the protein substrate have been determined. Several changes were experienced in the composition of microbe communities developing in fermentors that efficiently produce biogas from basic protein materials. In addition to significant identical features and similarities, differences were also found in the composition of the microbe community of the fermentors utilizing the two types of protein. Nevertheless, the common elements can also be used in general for promoting the adaptation of biogas plants utilizing protein-rich basic materials.

Instead of the application of metagenomic approaches, the species or the presence of particular species sought can also be determined with conventional methods or by utilizing species- specific markers [see e.g. Park et al., The Journal of Microbiology, June 2003, p.73-77]. However, this can easily become a more time consuming procedure.

Consequently, proteins can be used for the production of biogas, and for this a metagenomic evidence has been provided by the presentation of several protein-degrading microorganisms which are capable of adapting the system for high amounts of protein.

Below the invention is illustrated by specific experimental examples, however, the examples should not be construed as limiting the invention, since those skilled in the art will recognize that a number of variants and embodiments of the invention exist, in which the inventive idea is present.

### EXAMPLES DEMONSTRATING THE PRACTICE OF THE INVENTION.

### Example 1 Production of adapted activated sludge in batch fermentors

The fermentation was performed in 0.5-1 hypovial flasks. Adaptation was initiated by the addition of 0.5 g casein (SIGMA Casein, from bovine milk C7078-1KG CAS: 9000-71-9) to a fermented activated sludge (Agrospeciál Kft. Dunaújváros-Pálhalma 2407) containing 400-ml of methanogenic culture. After air-tight sealing the 100 ml aerial volume was anaerobized for 5 minutes. Fermentors were kept in incubators adjusted to 37°C. For the assessment of organic content, total carbon, total nitrogen and organic carbon, sample collected on day 5 prior to the addition of the subsequent portion of protein. For measuring enzyme activity samples were taken 30 minutes after the addition of casein. Subsequently, next portions of protein were added in each 5th day. The oTS (organic dry material content) according to the VDI standard (Verein Deutcher Ingenieure, 2006) is 1.5% of the reactor volume, and to avoid the event of substrate inhibition the recommended ratio between substrate oTS and inoculator sludge oTS is 0.5 The fermentation was initiated with a standard 0.5 g amount of casein which was later increased significantly.

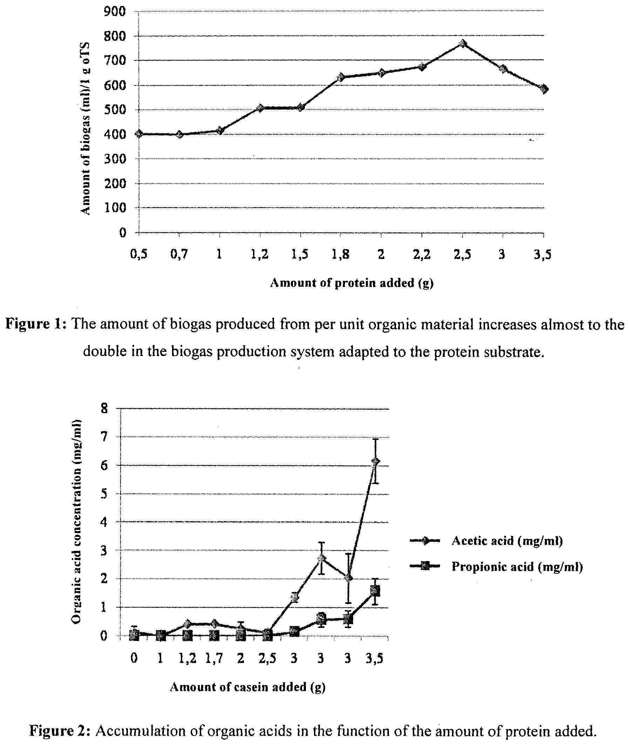

During the first 75 days 0.5 g casein was added to the inoculator sludge every 5th day. After this, the amount of protein added was elevated as it is summarized in figure 1. Initially due to the start of the slow adaptation it was not necessary to repeat the addition of the amounts, therefore the amount of protein added was not increased. The 0.5-1 batch adaptation studies are proved to be reproducible.

### Measuring organic acid activity

Among organic acids the concentration of acetic acid and propionic acid were measured by utilizing the already known methods. During adaptation it was observed that when the amount of fed substrate exceeded a certain limit the organic acid content started to increase. Therefore it is necessary to keep the amount of the fed substrate below this limit. In the given system the limiting value was 3 g, thus in each feeding the maximum amount was 2.5 g protein, i.e. 5 g/l reactor volume. This is 5 times higher than the optimal value of the VDI standard.

### Protease activity measurement

In the samples specific protease activity was measured with asocasein. Basically the asocasein is a modified form of casein used for the acclimatization, where a dye was bound to the basic protein. Following the protease cleavage a colored product appears in the solution, the amount of which can be measured spectrophotometrically at 440 nm. Extinction measured at this wavelength is proportional with the protease activity. In the sample preparation the samples were filtered through a 0.2 µm pore size sterile syringe filter. 200 µl sample was buffered with 50 µl K₂HPO₄-KH₂PO₄ buffer (pH 8) and after mixing 200 µl asocasein was added to the mixture and the samples were incubated for 24 hours at 37 °C. This period of time is long enough for the enzymes to perform the digestion. The reaction was stopped with the addition of 5% tricloric acid which precipitates every protein in the system, such as the enzymes and all non digested asocasein molecules, however the stain remains in solution and the intensity of the stain color is proportional to the enzymes present in the sample. Samples were centrifuged at 13000 rpm for 10 minutes, than the light absorption was measured spectrophotometrically at 440 nm. When the amount of protease enzymes elevates, a higher absorption is measured.

### Results

**Table 1: The amount of casein fed and the number of its repetitions during fermentation**

| Amount of fed casein (g/400 ml) | Batch repetition number |
|---|---|
| 0.5 | 15 |
| 0.7 | 2 |
| 1 | 4 |
| 1.2 | 2 |
| 1.5 | 1 |
| 1.8 | 1 |
| 2 | 1 |
| 2.2 | 1 |
| 2.5 | 1 |
| 3 | 1 |
| 3.5 | 1 |
| 4 | 1 |
| 4.5 | 1 |
| 5 | 1 |

Figure 1 shows the mean volume (ml) of biogas produced from 1 gram casein during batch fermentation. The x axis denotes the amount of casein added in grams, while the y axis denotes the volume of biogas produced from 1 gram of the protein added to the system. It is clearly shown that by adapting to the increased amount of protein added during the process of adaptation the microbes can utilize better the casein and produce more biogas from an unit amount.

Figure 2 shows the concentration of the two organic acids, the acetic acid and the propionic acid (mg/ml). The figure shows that after the addition of 3 grams of casein the organic acid content of the system started develop quickly leading to a system breakdown.

Figure 1 and 2 shows that an amount of 2.5 grams of protein (5 times higher than the optimum value of the standard) did not cause problems yet in the fermentation, however an amount exceeding this value resulted a quick increase in the organic acid content leading to the breakdown of biogas production.

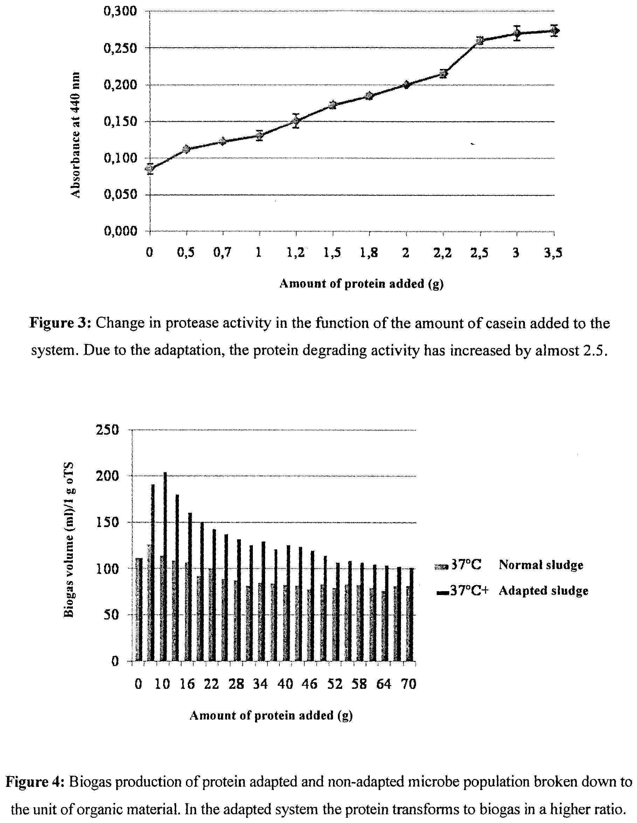

Figure 3 shows the increase in enzymatic activity during adaptation. By increasing the amount of protein added the activity, i.e. the amount of protein degrading enzymes produced by microbes, gradually elevates in the system.

### EXAMPLE 2 Use of adapted activated inoculator sludge for decomposition of swine blood in batch fermentors

The blood decomposition studies were also set up in batch fermentors. The protein decomposition and biogas production were performed with both completely fermented inoculator sludge containing normal, non adapted microbes and both with an inoculator sludge adapted earlier and containing casein. In the figure, the x axis contains the amounts of added blood is given in grams, while the y axis shows the volume of biogas produced in milliliters. Figure 4 clearly shows that compared with inoculation performed with sludge (control) the biogas yield increased significantly in samples containing adapted sludge. The example also demonstrates that due to the effects of adapted microbes the process of biogas production accelerated and the volume of biogas produced increased.

Table 2 shows the ratio of biogas intensification and the multiplication rate of the amount of blood added to the standard recommendation.

**Table 2 Increase in biogas volume produced from blood proteins and the increase in efficiency given in numbers.**

| **Blood (g)** | **Standard required value** | **Biogas production intensification (%)** |
|---|---|---|
| 7 | 2.0x | 151 |
| 10 | 2.9x | 179 |
| 13 | 3.7x | 166 |
| 16 | 4.6x | 150 |
| 19 | 5.4x | 163 |
| 22 | 6.3x | 142 |
| 25 | 7.1x | 153 |
| 28 | 8.0x | 150 |
| 31 | 8.9x | 153 |
| 34 | 9.7x | 151 |
| 37 | 10.6x | 143 |
| 40 | 11.4x | 150 |
| 43 | 12.3x | 150 |
| 46 | 13.1x | 154 |
| 49 | 14.0x | 137 |
| 52 | 14.9x | 134 |
| 55 | 15.7x | 130 |
| 58 | 16.6x | 129 |
| 61 | 17.4x | 132 |
| 64 | 18.3x | 135 |
| 67 | 19.1x | 124 |
| 70 | 20.0x | 124 |

### EXAMPLE 3 Production of adapted activated inoculator sludge in 5-1 continuous fermentors

Since the continuous fermentation is an open system, therefore it can be controlled well. The addition of biomass and the removal of fermentation residue is continuous. In the case of constant basic material composition the amount of biogas produced remains unchanged. The benefits of this technology: the basic material is easy to stir and to remove, thus the process can be well automatized. It is primarily used in the practice in biogas plants where the aim is to produce as high biogas yield as possible. Parameters playing important roles in the process can be modeled well in laboratory scale reactors.

The continuous fermentation was carried out in a 6-1 fermentor manufactured uniquely specifically for this purpose. The effective volume of the reactor was 5 liters with a gas chamber of 1 liter above. The software controlling the fermentor is able to adjust the temperature and to record the redox potential, pH, and the passing gas volume in every 4th hour (6 sampling each day). The substrate biomass is stored in a storage tank, and depending on the desired staying time the system feeds the suitable amount to the fermentor at regular periods. A special spiral blade is applied to stir the mixture. The fermentor is equipped with a gas and a liquid sampling valve. During the total period of fermentation the pH and the temperature were maintained on a determined level, while the value of the redox potential was followed. A redox potential below -300 mV is the optimal value for microbe strains having a role in the biogas fermentation. When the value of the redox potential exceeded -300 mV the air chamber of the fermentors was flushed with inert nitrogen gas to maintain the suitable aerobicity. By the utilization of an electric heating jacket the automation maintained the temperature at a desired temperature of 37C°± 0.2°C.

The fermentors were filled with completely fermented inoculator sludge and at the given time the casein substrate was added. Thus the added protein was the only available nutrient - and biogas - source for the microbe community in the system. Table 3 summarizes the protein addition stages. The amount of casein added was increased only when the acetic acid results did not indicate that the system becomes instable.

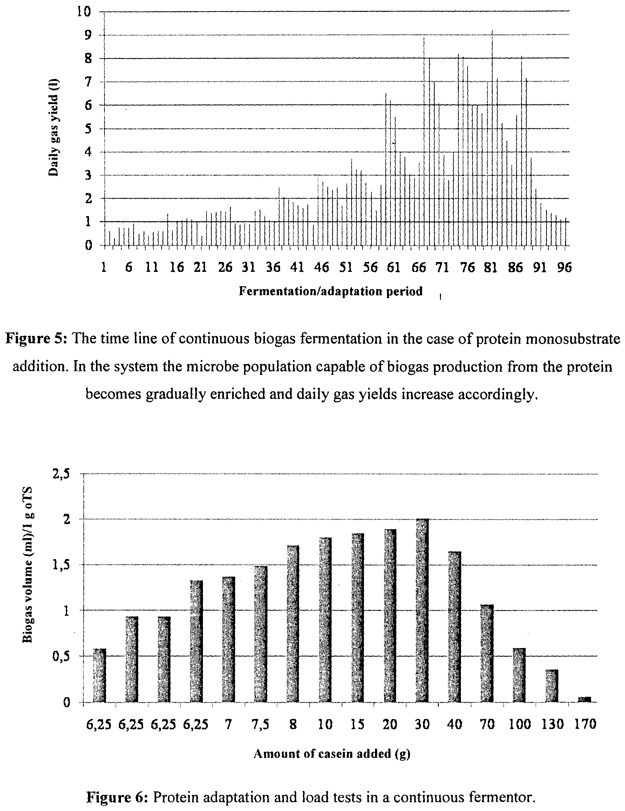

Figure 5 shows the increase in daily gas yield due to the elevated protein content in the adaptation period. Since the feeding was performed weekly, the biogas yield increased 1-2 days after the substrate addition, after that it decreased continuously.

Figure 6 shows the biogas yield produced in 1 gram protein as the function of the amount of casein added. As end product of adaptation, from 1 gram protein the microbes were capable of producing a gas yield 3.5 times higher than the initial value. The system reached the highest biogas yield at 30 gram of added protein. After this the amount of biogas produced form 1 gram casein was significantly reduced.

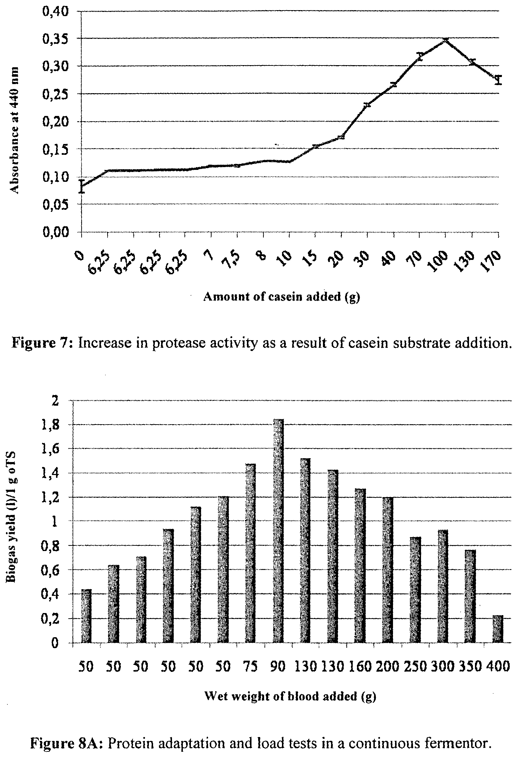

Figure 7 shows the mean enzyme activities as the function of the amount of casein added. Due to the increasing amount of protein the enzyme activity reached a value 5 times higher than at the initiation.

Clearly, maximum enzyme activity might be measured at 100 gram of added protein, however, this amount is significantly higher than the amount would be considered optimal on the basis of gas yield results. This may be explained by the fact that proteins added to the system are first converted to organic acids which will later be utilized by the microorganisms for their life functions and they produce methane from these acids. However, preeminently the hydrolyzing bacteria are responsible for the production of enzymes. The lower pH does not disturb the hydrolyzing bacteria, thus they are capable of maintaining their life functions in the presence of significant amount of organic acids. The biogas production limiting factors are the methanogenes, which do not work correctly at lower pH range.

Thus, while the environment is still suitable for the hydrolyzing bacteria and they still decompose the proteins with their enzymes, the conditions are far less optimal for the methanogenes to produce the biogas.

**Table 3**

| **Amount of fed casein (g/400 ml)** | **Stage repetition number** |
|---|---|
| 6.25 | 4 |
| 7 | 1 |
| 7.5 | 1 |
| 8 | 1 |
| 10 | 1 |
| 15 | 1 |
| 20 | 1 |
| 30 | 1 |
| 40 | 1 |
| 70 | 1 |
| 100 | 1 |
| 130 | 1 |

### EXAMPLE 4 Decomposition of swine blood in 5 1 continuous fermentors

Fresh untreated swine blood was used in this study. Clotted swine blood separated from the serum by decantation was frozen until use. Figure 8A shows the biogas yield produced from 1 g blood as a function of the amount of blood (g) added. The diagram clearly shows that the amount of produced biogas continuously increases until the amount of fed blood reaches 90 g, and then a gradual decrease can be observed in the biogas yield, which then drastically drops at the highest amount of blood (400 g).

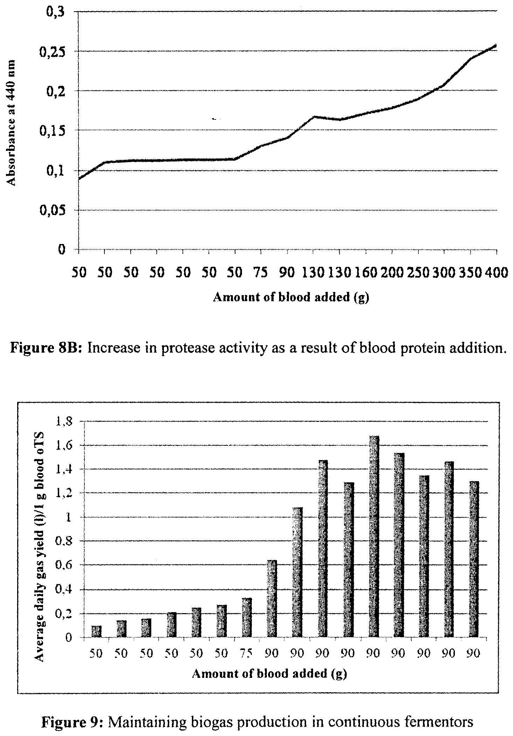

Figure 8B shows the absorbance proportional to the enzyme activity as a function of the amount of blood added. The situation is similar to that of for the measurement performed with casein fermentors. Although during the study the enzyme activity was gradually increasing due to the blood added, however the positive effect of this increase exerted on the gas yield could not be observed after a certain amount of blood has been added. The explanation of this event is similar to that of described for the casein adaptation.

### EXAMPLE 5 Maintaining biogas production in continuous fermentors

By repeating partially the study that demonstrated the decomposition of swine blood, our aim of this further fermentation was to test whether a long term stable biogas production can be maintained with protein monosubstrates or not. The adaptation was also performed as described above, however, when the maximum biogas yield for 1 g dry organic material was reached the amount of protein added has not been further increased, instead, this 90 g of substrate was fed weekly into the system for 2 months. Based on the results it can be concluded that the fermentation could be stabily maintained and the methane content of the biogas was between 62-70%. The experiment was continued for at least two months and during the period of the study the system did not show instability at all. Every week the amount of biogas not only reached the volume of 1000 ml/g dry organic material experienced in the previous study but it exceeded significantly in average. (Figure 9 shows the mean daily gas yield in liters, relative to 1 g of substrate, as the function of the amount of substrate added. Figure 3 shows the mean value of 3 independent, continuous fermentations in 5 1 volume. Values show a 15% deviation). It should be noted that silage corn, which is an excellent and widely used material for biogas, produces a yield of 600-650 ml/g organic dry material. Thus it can be concluded that by the utilization of protein rich starting materials the system is capable of producing significant and stably maintained biogas yield without being overloaded.

### EXAMPLE 6 Metagenomic analysis

During the adaptation experiments with swine blood and casein samples were collected for metagenomic analysis at certain periods (weekly) from the fermentors. DNA was purified from these samples for DNA sequence based identification of species forming the microbe community living in the fermentors. After obtaining DNA in suitable quality and quantity (m= 200-1000ng/µl; 260/280: 1.8-2.0) the samples originated from the blood or casein containing fermentors were sequenced in an Applied Biosystems SOLiD™ type sequencer. Since compared with the Sanger type sequencers it is capable of producing orders of magnitudes reads from the DNA , this system, being a new generation sequencer giving huge amount and reliable data, provides reliable result for the user. This reliability is ensured by the fact that DNA reading is based on ligation (the ligase enzyme ligates fluorescently labeled DNA probes identical with the complementary sequence of the DNA purified from the fermentor) and that each nucleotide is read two times. In the study, protein containing substrate taken at the inoculation (when the inoculator sludge was placed into the fermentor) and at 5 weeks post-inoculation was subjected to metagenomic analysis. Upon sequencing 25-35 million reads were generated which were assembled to longer contigs by using the CLC program directly developed for metagenomics works. The on-line available MG-RAST program compared these contigs, i.e. the assembled reads, with the following databases: GenBank, IMG, KEGG, PATRIC, RefSeq, SEED, SwissProt, TrEMBL, eggNOG, COG, KO, NOG, Subsystems, LSU, RDP, SSU. By the help of this the strains were determined taxonomically even at species level and known functions considered to be important (e.g. genes involved in the metabolism, etc.) were analyzed. The results were validated for "e" value and for percentage of identity. (The e value of less than 10⁻⁶ indicates a good reliability of the data, and describes the random background noise which depends on the length of sequence determined, and the closer the value to zero the accuracy of the identity is higher. The percentage of identity shows to what extent the determined sequence is identical with similar sequences found in the database, expressed in percentage. The value is accepted when the identity is higher than 70%) Several hundreds of microorganisms were identified after data screening.

In favour of accuracy of the analysis the non assembled readings were aligned with these species, since contigs generated by assembling each of the readings might contains errors, i.e. the contig may comprise erroneous readings, and the alignment of the given contig with the database could result an erroneous result, therefore the obtained results were subjected to further inspection. Data without assembling confirmed the original results well. Thus, this step might be theoretically omitted when there is a need for quick examination or when the method has already been adjusted.

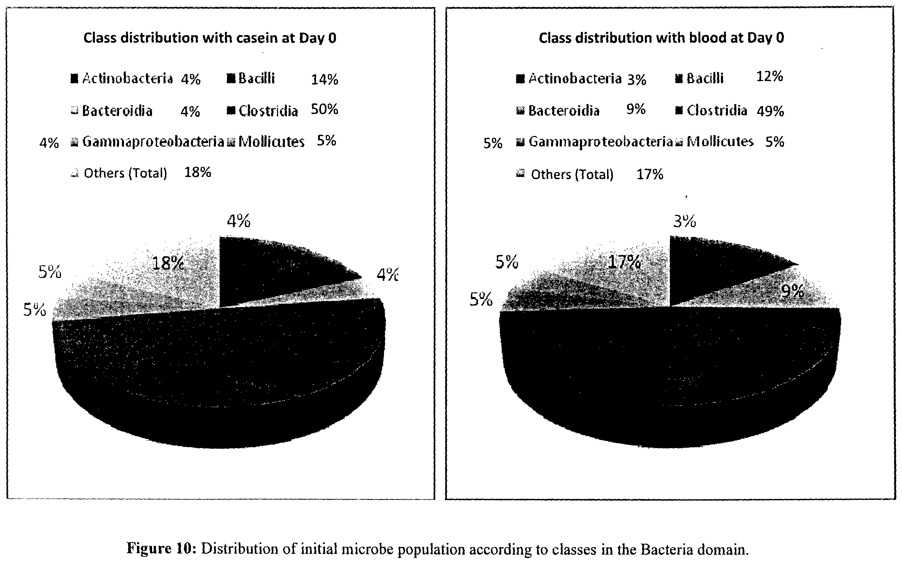

According to the data there is no significant difference between the microorganism composition of the blood and casein sample, the composition of the two fermentors is similar, the initial conditions correspond to the normal population composition- i.e. the population composition of protein-free fermentors - both in the Bacteria and both in the Archaea domains. This corresponds to the fact that at this time the system did not comprise proteins (Figure 10). Because sludge originated from a normal (fed with plantal basic material) biogas fermentor was used as inoculator sludge, and the microbe community of this was adapted to large amount of proteins.

### Results of metagenomic analysis

In the Bacteria domain the basic population distribution in both fermentor shows good agreement with the population composition of such fermentors known from the literature, where plantal substances were used as substrate [Schluter et al., J Biotechnol. 2008 Aug 31; 136(1-2):77-90.]. In biogas fermentors the plant biomass (silage corn, etc.) decomposes through different metabolic pathways by the work of microbes. As a first step the materials constituting the wall of the plant cell, such as cellulose and hemicellulose) decomposes to mono-, di- and oligosaccharids. This hydrolytic step is performed primarily by the *Clostridia* and *Bacilli* class species having cellulytic activity. The sugar intermediers are fermented to organic acids (acidogenesis), due to the secondary fermentation of the microbes acetate, CO₂, and H₂ are formed from the sugars. The last step is the methanogenesis that is performed by the *Archaea* species. In the fermentor studied the species belonging to the Clostridia (49-50%) and Bacilli (12-14%) classes prevail. Not only the distribution percentage but the species of the other class are mainly identical, i.e. the composition of the initial population in the two fermentors is similar as it can be seen in the following table (Table 4).

**Blood, Day 0**

| **Species** | **Strain** | **Class** | **Order** | **Hit¹** |
|---|---|---|---|---|
| Slackia heliotrinireducens | Actinobacteria | Actinobacteria (class) | Coriobacteriales | 60 |
| Streptococcus pneumoniae | Firmicutes | Bacilli | Lactobacillales | 85 |
| Enterococcus faecium | Firmicutes | Bacilli | Lactobacillales | 88 |
| Enterococcus faecalis | Firmicutes | Bacilli | Lactobacillales | 128 |
| Bacteroides fragilis | Bacteroidetes | Bacteroidia | Bacteroidales | 95 |
| Bacteroides sp. | Bacteroidetes | Bacteroidia | Bacteroidales | 97 |
| Thermoanaerobacter sp. X514 | Firmicutes | Clostridia | Thermoanaerobacterales | 101 |
| Pelotomaculum thermopropionicum | Firmicutes | Clostridia | Clostridiales | 121 |
| Clostridium cellulolyticum | Firmicutes | Clostridia | Clostridiales | 125 |
| Clostridium kluyveri | Firmicutes | Clostridia | Clostridiales | 143 |
| Syntrophomonas wolfei | Firmicutes | Clostridia | Clostridiales | 155 |
| Finegoldia magna | Firmicutes | Clostridia | Clostridiales | 183 |
| Alkaliphilus oremlandii | Firmicutes | Clostridia | Clostridiales | 184 |
| Desulfitobacterium hafniense | Firmicutes | Clostridia | Clostridiales | 219 |
| Caldanaerobacter subterraneus | Firmicutes | Clostridia | Thermoanaerobacterales | 226 |
| Clostridium difficile | Firmicutes | Clostridia | Clostridiales | 274 |
| Alkaliphilus metalliredigens | Firmicutes | Clostridia | Clostridiales | 277 |
| Clostridium thermocellum | Firmicutes | Clostridia | Clostridiales | 295 |
| Escherichia coli | Proteobacteria | Gammaproteobacteria | Enterobacteriales | 61 |
| Acholeplasma laidlawii | Tenericutes | Mollicutes | Acholeplasmatales | 240 |

**Kazeines 0 napos**

| **Species** | **Strain** | **Class** | **Order** | **Hit¹** |
|---|---|---|---|---|
| Bifidobacterium longum | Actinobacteria | Actinobacteria (class) | Bifidobacteriales | 50 |
| Streptococcus suis | Firmicutes | Bacilli | Lactobacillales | 75 |
| Enterococcus faecalis | Firmicutes | Bacilli | Lactobacillales | 145 |
| Bacteroides thetaiotaomicron | Bacteroidetes | Bacteroidia | Bacteroidales | 49 |
| Bacteroides vulgatus | Bacteroidetes | Bacteroidia | Bacteroidales | 72 |
| Clostridium saccharolyticum | Firmicutes | Clostridia | Clostridiales | 116 |
| Clostridium cellulolyticum | Firmicutes | Clostridia | Clostridiales | 125 |
| Clostridium beijerinckii | Firmicutes | Clostridia | Clostridiales | 136 |
| Caldicellulosiruptor | Firmicutes | Clostridia | Thermoanaerobacterales | 138 |

**Table 4: The 20 most abundant species occurring at the start in the blood and casein fermentors 1.: Number of hits on the assembled contig**

| | | | | |
|---|---|---|---|---|
| saccharolyticus | | | | |
| Clostridium kluyveri | Firmicutes | Clostridia | Clostridiales | 155 |
| Desulfotomaculum reducens | Firmicutes | Clostridia | Clostridiales | 175 |
| Syntrophomonas wolfei | Firmicutes | Clostridia | Clostridiales | 176 |
| Pelotomaculum thermopropionicum | Firmicutes | Clostridia | Clostridiales | 185 |
| Finegoldia magna | Firmicutes | Clostridia | Clostridiales | 197 |
| Alkaliphilus metalliredigens | Firmicutes | Clostridia | Clostridiales | 270 |
| Clostridium difficile | Firmicutes | Clostridia | Clostridiales | 271 |
| Desulfitobacterium hafniense | Firmicutes | Clostridia | Clostridiales | 298 |
| Clostridium thermocellum | Firmicutes | Clostridia | Clostridiales | 298 |
| Escherichia coli | Proteobacteria | Gammaproteobacteria | Enterobacteriales | 46 |
| Acholeplasma laidlawii | Tenericutes | Mollicutes | Acholeplasmatales | 197 |

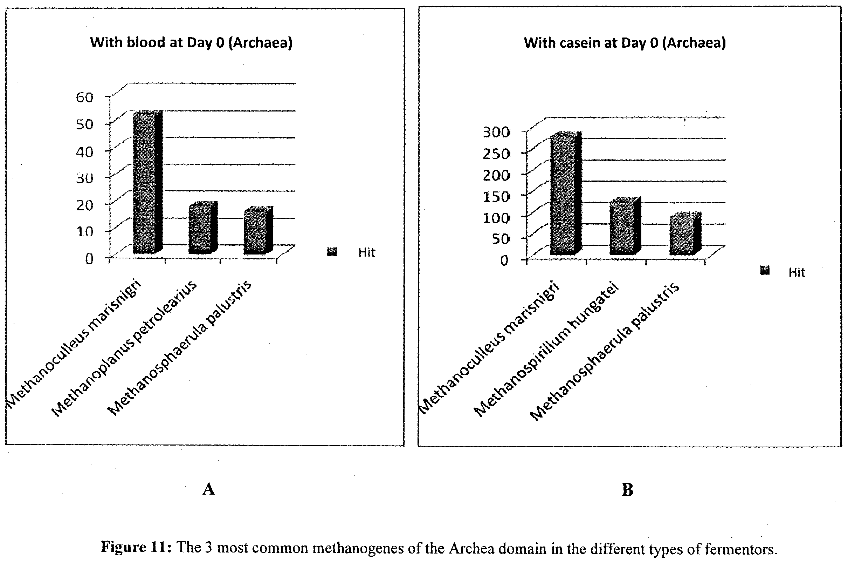

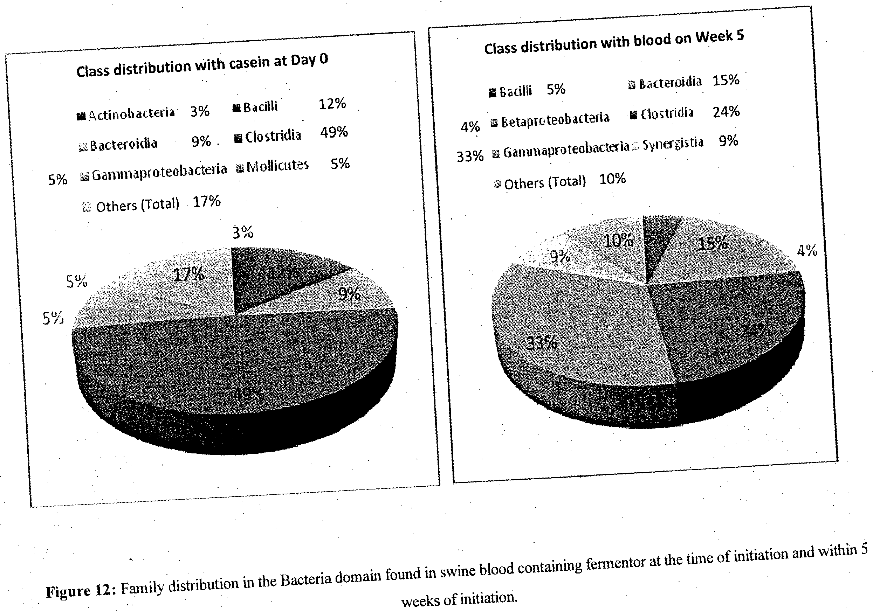

Species belonging to the *Actinobacteria* family were found in both fermentors. These species occur in huge amount in the soil and the natural waters. They have significant role in decomposition of such organic materials as the cellulose. In the fermentors studied two species of the *Actinobacteria* family - the *Slackia heliotrinireducens,* and *Bifidobacterium longum -* were found in the highest amount. The *Slackia heliotrinireducens* is a Gram positive, anaerobe bacterium which capable of reducing nitrate to ammonia provided as electron donor (H₂, formate) is present in the system, furthermore due to its metabolic activity this bacterium is also able to produce acetic acid and propionic acid [Pukall et al. Stand Genomic Sci. 2009 Nov 22; 1(3):234-41.]. The *Bifidobacterium longum* is a Gram positive bacterium which can also be a part of the normal human bowel flora. As it is characteristic to the members of the *Actinobacteria* family this species degrades the hardly digestible plant polymers to simple sugars in the bowel [Sela et al. Proc Natl Acad Sci USA. 2008 Dec 2; 105(48):18964-9.] Mostly (49-50%) the members of *Clostridia* family were found in the fermentors. Within this family mainly the *Clostridium thermocellum* occurred. With the help of its high number of extracellular cellulases this species is capable of degrading the cellulose [Zverlov et al. Proteomics. 2005 Sep; 5(14):3646-53.], from which it produces ethanol. Besides C. *thermocellum,* high amount of C. *difficile, C. cellulolyticum, C. saccharolyticum, C. kluyveri* species were found, which also show cellulytic capacity. Besides these species other *Clostridium* species were also found. The *Desulfitobacterium hafniense* is an anaerobe cellulose fermenting species producing acetate and ethanol from cellulose [Nonaka et al. J Bacteriol. 2006 Mar; 188(6):2262-74.]. The *Syntrophomonas wolferi* is a microbe fermenting long chain fatty acids and which is able to propagate in co-cultures with methanogenic *Archaea-s* [McInerney et al. Appl Environ Microbiol. 1981 Apr; 41(4):1029-39.]. *Pelotomaculum thermopropionicum* species were also found in high quantity. Similarly to *Syntrophomonas wolferi* this species lives in syntrophy with the methanogenes and the metabolically different microbes are able to exist by helping each other. This syntropic connection has important role in the conversion of organic material to methane [Schink et al. Microbiol Mol Biol Rev. 1997 Jun; 61(2):262-80.]. Two metal reducing bacteria - *Alkaliphillus methalliredigens* and *Desulfotomaculum reducens -* were also found. For the reduction of iron and cobalt these species use lactate and acetate as electron donor [Ye et al. Appl Environ Microbiol. 2004 Sep; 70(9):5595-602.]. The *Caldicellulosiruptor saccharolyticus* is a hydrogen producing bacterium with cellulytic activity. When *C. saccharolyticus* is added to waste sludge or dried plant biomass the biogas production increases significantly [Bagi et al. Appl Microbiol Biotechnol. 2007 Aug; 76(2):473-82.] The high number of *Clostridia*species may suggest that this family plays important role in the normal microbe flora of fermentors fed with plantal substrate. Due to their cellulose degrading activity they provide simpler substrates for other microbe species, additionally the member of this family is known to be capable of performing secondary fermentation - i.e. degrading simple sugars [Demain et al. Microbiol. Mol. Biol. Rev. 69, 124-154.] thus preparing nutrients for the Archaea-s and facilitating biogas production. The second largest family is the *Bacilli* family. Among its members the *Enterococcus faecalis* can be found in the highest amount. This is an anaerobe Gram positive species living in the bowel and capable of degrading plant polysaccharides [van Schaik et al. BMC Genomics. 2010 Apr 14; 11:239.]. Besides the already mentioned *Clostridia* and *Bacilli* families, high amount of *Bacteroidia* species were also found in the soil and sediment. The members of this family are also capable of fermenting simple sugars, although not these compounds but also the polysaccharides provide the most significant energy source for them. Certain members of this family are capable of binding nitrogen. *Bacteroidia* species found are quite common in putrescent materials. Members of the *Mollicutes* family are capable of living in both anaerobe and both aerobe way. Anaerobe representatives of this family are capable of producing organic acids [Maniloff, ASM Press, 1992]. In the most known genus of the *Mollicutes* family, the *Acholeplasmatales* genus, the *Acholeplasma laidlawii* species was found, which is capable of fermenting glucose to saturated fatty acids, lactic acid and acetate. Among species belonging to the *Gammaproteobacteria* family the *Escherichia coli (Enterobacteriales)* was found in high numbers. The *E. coli* is a Gram negative, facultative anaerobe bacterium which utilizes different acids for producing succinate, ethanol, acetate and carbon dioxide. In the fermentation of acids hydrogen is produced, therefore this species is an important symbiont of the methanogenes or the sulfate reducing bacteria. The *Archaea-s* are responsible for methane production. The results clearly show that considering methane production the *Methanomicrobiales* order plays important role in these fermentors since the most abundant four species belong to this order. The four most abundant species in the two fermentors are the *Methanoculleus marsinigri, Methanoplanus petrolearius, Methanospirillum hungatei,* and the *Methanosphaerula palustris.* (Figures 11A and 12B)

A *Methanoplanus petrolearius* plays important role in carbon cycling. This species can utilize CO₂ and H₂ as a source of energy. The acetate is necessary for growth and as carbon source [Brambilla et al. Stand Genomic Sci. 2010 Oct 27; 3(2):203-11.]. The *Methanospirillum hungatei* is a hydrogenotrophic methanogene (this characteristical for the *Methanomicrobiales* genus), i.e. for the production of methane it utilizes H₂ as electron source to reduce CO₂. This species grows in a chain consisting of 9-12 cells. For growing it needs acetate and pyruvate [Qiu et al. Appl Environ Microbiol. 2004 Mar; 70(3):1617-26.] In both fermentors the third most abundant species is the *Methanosphaerula palustris* which is also a hydrogenotrophic methanogene that needs acetate for growing [Cadillo-Quiroz et al. Int J Syst Evol Microbiol. 2009 May; 59(Pt 5):928-35.]. Extremely high amount of *Methanoculleus marsinigri* (*Methanoculleus* genus) was found. The *M. marsinigri* is also a hydrogenotrophic methanogenic species. It reduces the carbon dioxide to methyl then by the catalysis of its methyl coenzyme reductase (this enzyme is characteristic to the methanogene [Zhu et al. Microbiol Res. 2011 Jan 20; 166(1):27-35.]) converts the methyl to methane. For methanogenesis this species is also capable of utilizing secondary alcohols as electron donor. The fact that the *Methanoculleus marsinigri* can be found in extraordinary high number in the initial fermentors and that the *Methanomicrobiales* order plays important role in the methanogenesis at this point meet those published data [Schlüter et al. J Biotechnol. 2008 Aug 31; 136(1-2):77-90., Krause et al. J Biotechnol. 2008 Aug 31; 136(1-2):91-101.] which deal with composition of the normal, i.e. not protein adapted biogas producing population of microorganisms.

Samples collected at week 5 after initiation were used for further metagenomic analyses and compared with previous results. The inventors experienced that compared with the initial situation the composition of populations in the samples obtained at week 5 showed differences in both the *Archaea* (in samples containing swine blood) and in both in the *Bacteria* domain. However, the changes in *Bacteria* domain were the most spectacular. According to our observation, after starvation the microbe population grew on plantal substrate is capable of degrading the swine blood and casein as well as producing biogas. So that it become possible the microbe population was adapted specially for degrading the given protein in the fermentor.

### A) Analysis of microbial community present in fermentors containing swine blood

The adaptation to proteins can be tracked by analyzing species compositions in each fermentors. Regarding the distribution, the members of *Actinobacteria, Bacilli* and *Mollicutes* family of the *Bacteria* domain occupy a lower ratio, while species belonging to the *Bacteroidia, Synergistia* and *Beteproteobacteria* families occur in a higher ratio in the blood containing fermentor. The number of *Gammaproteobacteia* species increased considerably while less *Clostridia* was observed (**Figures 12A and 12B**).

The high amount of protein appearing in the fermentor may be reason of the decrease in *Clostridia* species. Families capable of fermenting protein and metabolizing blood come into the foreground. It can be excellently followed on the figure above. Representatives of the *Clostridia* family diminish significantly against the ***Gammaproteobacteria,*** and the number of *Synergistia, Betaproteobacteria, Bacteroidia* species increases. The species belonging to *Clostridia* family are mainly the same as found at initiation (Table 5). The *Clostridium thermocellum* having cellulytic activity is still present in high amount. However, the amount of *Alkaliphilus metalliredigens* increased significantly in the fermentor. This species is capable of reducing not only the iron and cobalt [Ye Q. et al: Appl Environ Microbiol. 2004 Sep; 70(9):5595-602.] but the selenium and manganese as well [Yul Roh et al: Alkaliphilus metalliredigens (QYMF), 11(4):415-423 (2007)]. It utilizes the above mentioned acetate and lactate as well as the hydrogen and yeast extract as electron donor. Possibly the high amount of iron which was introduced with the swine blood would explain the proliferation of this species. Similarly, in the community cultivated on casein the selenium may induced their spreading.

**Table 5: Composition of population in the swine blood containing fermentor on week 5 after initiation.**

| **Species** | **Strain** | **Class** | **Order** | **Hit¹** |
|---|---|---|---|---|
| Enterococcus faecium | Firmicutes | Bacilli | Lactobacillales | 71 |
| Enterococcus faecalis | Firmicutes | Bacilli | Lactobacillales | 105 |
| Bacillus subtilis | Firmicutes | Bacilli | Bacillales | 199 |
| Parabacteroides johnsonii | Bacteroidetes | Bacteroidia | Bacteroidales | 92 |
| Porphyromonas gingivalis | Bacteroidetes | Bacteroidia | Bacteroidales | 212 |
| Bacteroides thetaiotaomicron | Bacteroidetes | Bacteroidia | Bacteroidales | 259 |
| Bacteroides vulgatus | Bacteroidetes | Bacteroidia | Bacteroidales | 274 |
| Bacteroides fragilis | Bacteroidetes | Bacteroidia | Bacteroidales | 367 |
| Parabacteroides distasonis | Bacteroidetes | Bacteroidia | Bacteroidales | 371 |
| Bacteroides sp. | Bacteroidetes | Bacteroidia | Bacteroidales | 468 |
| Bordetella avium | Proteobacteria | Betaproteobacteria | Burkholderiales | 60 |
| Bordetella bronchiseptica | Proteobacteria | Betaproteobacteria | Burkholderiales | 62 |
| Bordetella pertussis | Proteobacteria | Betaproteobacteria | Burkholderiales | 66 |
| Achromobacter xylosoxidans | Proteobacteria | Betaproteobacteria | Burkholderiales | 77 |
| Clostridium cellulolyticum | Firmicutes | Clostridia | Clostridiales | 151 |
| Halothermothrix orenii | Firmicutes | Clostridia | Halanaerobiales | 156 |
| Finegoldia magna | Firmicutes | Clostridia | Clostridiales | 167 |
| Caldicellulosiruptor saccharolyticus | Firmicutes | Clostridia | Thermoanaerobacterales | 182 |
| Carboxydothermus hydrogenoformans | Firmicutes | Clostridia | Thermoanaerobacterales | 205 |
| Clostridium kluyveri | Firmicutes | Clostridia | Clostridiales | 213 |
| Pelotomaculum thermopropionicum | Firmicutes | Clostridia | Clostridiales | 249 |
| Clostridium perfringens | Firmicutes | Clostridia | Clostridiales | 262 |
| Alkaliphilus oremlandii | Firmicutes | Clostridia | Clostridiales | 265 |
| Desulfitobacterium hafniense | Firmicutes | Clostridia | Clostridiales | 304 |
| Clostridium difficile | Firmicutes | Clostridia | Clostridiales | 384 |
| Clostridium thermocellum | Firmicutes | Clostridia | Clostridiales | 454 |
| Alkaliphilus metalliredigens | Firmicutes | Clostridia | Clostridiales | 459 |
| Pseudomonas amygdali | Proteobacteria | Gammaproteobacteria | Pseudomonadales | 93 |
| Escherichia coli | Proteobacteria | Gammaproteobacteria | Enterobacteriales | 173 |
| Pseudomonas entomophila | Proteobacteria | Gammaproteobacteria | Pseudomonadales | 222 |
| Pseudomonas savastanoi | Proteobacteria | Gammaproteobacteria | Pseudomonadales | 266 |
| Azotobacter vinelandii | Proteobacteria | Gammaproteobacteria | Pseudomonadales | 293 |
| Pseudomonas syringae group genomosp. 3 | Proteobacteria | Gammaproteobacteria | Pseudomonadales | 300 |
| Pseudomonas mendocina | Proteobacteria | Gammaproteobacteria | Pseudomonadales | 423 |
| Pseudomonas stutzeri | Proteobacteria | Gammaproteobacteria | Pseudomonadales | 459 |
| Pseudomonas syringae | Proteobacteria | Gammaproteobacteria | Pseudomonadales | 630 |
| Pseudomonas putida | Proteobacteria | Gammaproteobacteria | Pseudomonadales | 707 |
| Pseudomonas aeruginosa | Proteobacteria | Gammaproteobacteria | Pseudomonadales | 893 |
| Pseudomonas fluorescens | Proteobacteria | Gammaproteobacteria | Pseudomonadales | 973 |
| Dethiosulfovibrio peptidovorans | Synergistetes | Synergistia | Synergistales | 314 |
| Thermanaerovibrio acidaminovorans | Synergistetes | Synergistia | Synergistales | 326 |
| Aminobacterium colombiense | Synergistetes | Synergistia | Synergistales | 330 |
| Anaerobaculum hydrogeniformans | Synergistetes | Synergistia | Synergistales | 1068 |

### 1.: Number of hits on the assembled contig

Regarding species distribution the ratio of bacteria belonging to the *Bacteroidia* family increased from 9% to 15%. As it was described above, the members of this family can metabolize different polysaccharides. Among the members of this family the *Parabacteroides distasonis* ferments acetate, cellobiose, glucose, lactose and mannose. The *Bacteroides fragilis, B. vulgatus, B. thetaiotaomicron* a species can be found in bowel flora, and they also utilize different carbohydrates [Cerdeño-Tárraga AM, et al: Science. 2005 Mar 4; 307(5714):1463-5; McCarthy RE, et al: Appl Environ Microbiol. 1988 Aug; 54(8):1911-6.; Falony G, et al: Appl Environ Microbiol. 2009 Apr; 75(8):2312-9. Epub 2009 Feb 27; Gibson and Macfarlane, J. Gen. 134: 19-27.] The *B. fragilis,* and *B. vulgatus* are capable of growing also on blood agar [Cuív PÓ, et al: J Bacteriol. 2011 Aug; 193(15):4025-6. Epub 2011 May 27]. Among species belonging to the *Betaproteobacteria* family the Gram negative *Achromobacter xylosoxidans* species was found in the highest number. With the help of its oxydase and catalase this species is capable of oxidizing glucose and xylose. Besides oxidation it is capable of reducing nitrate to nitrite. This species frequently occurs also in urine and blood [Y Igra-Siegman, et al: J Clin Microbiol. 1980 February; 11(2): 141-145] Among Gram-negative pathogen coccus *Bordatella* species *Bordatella pertussis, Bordetella bronchiseptica* and *B. avium* species having hemolytic activity were found [Bellalou J, et al: Infect Immun. 1990 Oct; 58(10):3242-7; Jacob-Dubuisson F, et al: Microbiology. 2000 May; 146 (Pt 5):1211-21; Kirby AE, et al: Infect Immun. 2001 Nov; 69(11):6951-61.], thus during blood fermentation these species seemed to be commonly occurring. Among species belonging to the *Gammaproteobacteria* family, members of the *Pseudomonadales* genus were found in the highest amount. The *Pseudomonas* species are common in the nature. These species possess proteolytic activity [Laura FRANZETTI*, Mauro SCARPELLIN: Annals of Microbiology, 57 (1) 39-47 (2007)]. The pathogen *Pseudomonas aeruginosa* uses various extracellular enzymes which may contribute to infection by this species [Stepińska MA,et al: Curr Microbiol.2010 May; 60(5):360-4. Epub 2009 Dec 3 ]. Such proteolytic enzymes are the alkaline protease and elastase and with these enzymes this species is capable of degrading of elastine, collagene and proteoglycans. P. *aeruginosa* is also capable to degrade the haemoglobin [Vermelho AB et al: Mem Inst Oswaldo Cruz. 1996 Nov-Dec; 91(6):755-60]. The *Pseudomonas fluorescens* may participate in the process of blood clotting [Gibb AP, et al: J Clin Pathol. 1995 Aug; 48(8):717-8]. It contains heat stable proteases and lipases. The naturally highly abundant *Pseudomonas putida* also has different metabolic activity. In natural environment it is capable of removing the xylane and toluol contaminations [Yu H, et al: Biodegradation. 2001; 12(6):455-63]. It occurs frequently on decomposing plantal substrates [Nelson KE, et al: Environ Microbiol. 2002 Dec; 4(12):799-808]. The *Pseudomonas stutzeri* is a denitrificating species which can utilize the ammonium ion and the nitrate, furthermore it needs only simple organic molecules for growing. Due to this property it has significant role in the protein containing fermentors, i.e. it is capable of removing the ammonium ion originated from the high nitrogen content of the protein from the system [Jorge Lalucat, et al: Microbiol Mol Biol Rev. 2006 June; 70(2): 510-547]. Blood lysing *Pseudomonas mendoncina* was also found in high quantity in fermentors containing swine blood. For growth this species needs available xylose and glucose [Aragone MR, et al: J Clin Microbiol. 1992 Jun; 30(6):1583-4]. Similarly to the *Pseudomonas stutzeri* the *Azotobacter vinelandii* is a nitrogen fixing bacterium which can convert N₂ to NH₃ by the help of its nitrogenase Besides the nitrogenase it also has another oxygene sensitive enzyme, such as the formate dehydrogenase [Setubal JC, et al: J Bacteriol. 2009 Jul; 191(14):4534-45. Epub 2009 May 8]. When this species lives in aerobe environment by the help of its iron containing superoxide dismutase it is capable of converting the superoxid radicals produced by the cell itself to hydrogen peroxide and oxigene, and it protects its oxygene sensitive enzymes with alginate. In anaerobe environment it utilizes NADH and succinate as electron source [Qurollo BA, et al: Can J Microbiol. 2001 Jan; 47(1):63-71]. The *Pseudomonas entomophila* is a close relative of the *P. putida* species. Due its various metabolic activity this species is capable of colonization on different living places such as on decomposing materials. It secretes several types of degrading enzymes, e.g. proteases and lipases It also has haemolytic activity [Vallet-Gely I, et al: Appl Environ Microbiol. 2010 Feb; 76(3):910-21. Epub 2009 Dec 18]. Significant number of species belonging to the *Synergistia* family were found. The members of this family are Gram positive, anaerobe bacteria which are able to grow in several types of living places. These species are capable of metabolizing amino acids [Vartoukian SR, et al: The division "Synergistes", Anaerobe. 2007 Jun-Aug; 13(3-4):99-106. Epub 2007 Jun 7]. Among members of this family the *Anaerobaculum hydrogeniformans* species was found in the highest amount. The *A. hydrogeniformans* utilizes different sugars, such as maltose, glucose, fructose and mannose, from which it produces high amount of hydrogen and acetate. It is also capable of metabolizing certain amino acids, such as asparagine, cisteine, histidine, leucine, serine, phenylalanine, treonine and valine. It can also grow on tryptone and yeast extract [Maune MW and Tanner RS : Int J Syst Evol Microbiol.2011 May 20] Anaerobaculum hydrogeniformans sp. nov., a novel anaerobe that produces hydrogen from glucose. In the presence of pyruvate the *Aminobacterium colombiense* ferments serine, glycine and treonine amino acids. Its fermentation end product is propionate and hydrogen [Chertkov O, et al: Stand Genomic Sci. 2010 Jun 15; 2(3):280-9]. The *Termanaerovibrio acidaminovorans* converts by fermentation glutamate, histidine, ornitine, lysine and threonine into acetate and propionate; serine, pyruvate, glucose, fructose, xylose, glycerine into acetate and the branched chain amino acids into branched chain fatty acids. It is also capable of producing hydrogen which is an important substrate for the hydrogenetrophic *Methanothermobacter thermoautotropicum* living in co-culture with this species [Chovatia M,et al: Stand Genomic Sci. 2009 Nov 22; 1(3):254-61] The Gram negative *Dethiosulfovibrio peptidovorans* Gram negative is a distant relative of the *Thermanaerovibrio* genus. This species is an anaerobe thiosulphate reducing bacterium which can utilize peptides and amino acids as well as different sugars and fatty acids [Labutti K, et al: Stand Genomic Sci. 2010 Aug 20; 3(1):85-92] Amino acid metabolizing microbe was also found in the family of *Clostridia.* Besides utilizing different sugars the C. *perfingens* can ferment the serine and threonine to propionate and its fermentation end product are the acetate, ethanol, butyrate, lactate as well as the CO₂ and H₂ [Shimizu T,et al: Proc Natl Acad Sci U S A.2002 Jan 22; 99(2):996-1001. Epub 2002 Jan 15] In blood-containing fermentors the most abundant family is the *Gammaproteobacteria.* Similarly to the haemolytic *Betaproteobacteria* species, among the members of this family, that have haemolytic, proteolytic and denitrifying properties primarily the ones with haemolytic as well as denitrifying property play important role in the blood decomposition. This feature is well demonstrated by the fact that the representatives of these families proliferated significantly compared to the initial status. The amino acid fermenting property of the *Synergistia* family also demonstrates the adaptation to proteins, namely during the fermentation of amino acids originated from proteins decomposed by the *Beta-* and *Gammaproteobacteria* species, simpler molecules got in the nutrient circulation thus promoting the life and survival of other species and finally the conversion of the energy originated from protein sources to methane.

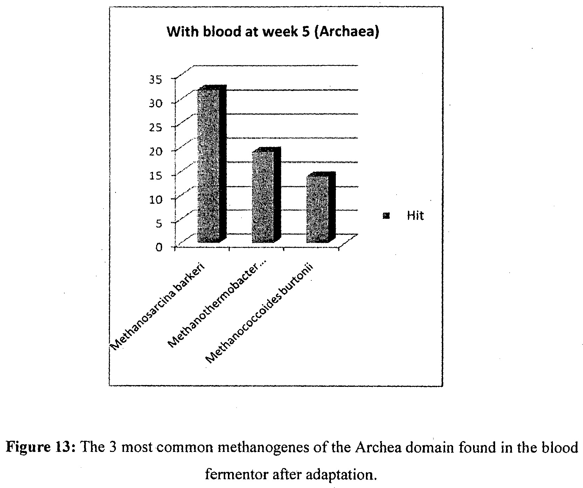

In the *Archaea* domain the most spectacular change occurred in the blood samples. Other metanogenes replaced the *Methanoculleus marsinigri.* (Figure 13) The *Methanosarcina barkeri* is a methylotrophic methanogene, i.e. for methane production it utilizes H₂, CO₂, or instead of using formate or acetate it prefers methylamines or methanol [Metcalf WW, et al: J Bacteriol. 1996 Oct; 178(19):5797-802].

The *Methanotermabacter thermoautotrophicus* has already mentioned above. This species lives in syntrophy with *Termanaerovibrio acidaminovorans* which provides H₂ for it, i.e. this species is a hydrogenetrophic methanogenic species. *T. thermautotropicus* is able to tolerate the presence of higher level of ammonia and hydrogen peroxide [Kato S, et al: Environ Microbiol. 2008 Apr; 10(4):893-905. Epub 2007 Nov 25]. The *Methanococcides burtonii* also belongs to the methylotrophic methanogenic species. However, by having Calvin cycle modified for binding CO₂ and carbon dioxide dehydrogenase enzyme this species catalyzes the conversion of carbon monoxide to carbon dioxide which is then converted to methane by the help of methyl coenzyme M reductase [Goodchild A, et al: J Proteome Res. 2004 Nov-Dec; 3(6):1164-76].

### B) Analysis of microbial community present in fermentors containing casein

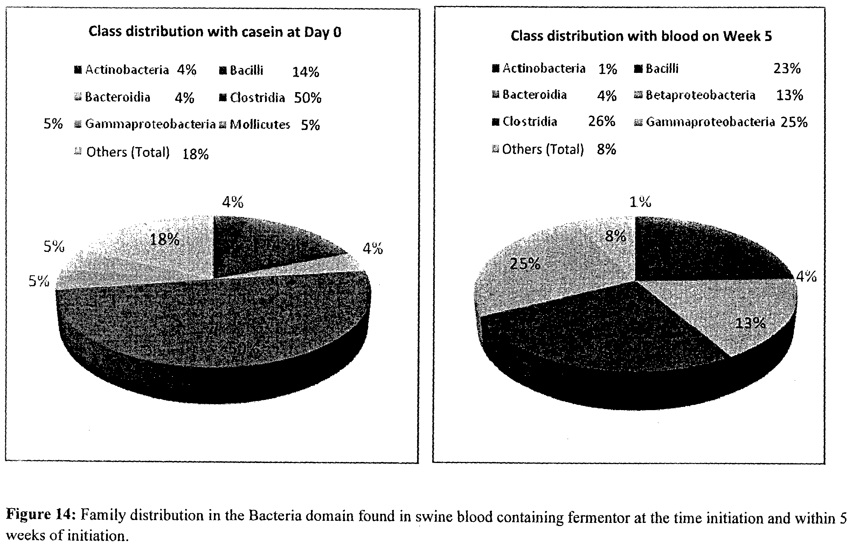

Parallel with samples from biogas fermentors containing swine blood as substrate the effect of casein as clean protein substrate on methane producing microbe community was studied. According to the results the casein lead to a community with partially different composition compared with the fermentor containing blood. The repression of *Clostridia* family was also observed in this case. However, the distribution between *Bacilli* and a *Gammaproteobacteria* is more consistent here, compared with the blood-containing fermentor. (Figures 14A and 14B)

Plantal substrate remaining in the fermentor and the sporing ability of the family members make possible for certain members of the *Clostridia* family to survive, however, the high amount of protein modifies the composition of the community. Similarly to the bloody samples also the *Alkaliphilus metalliredigens* species appear in high amount besides the *Clostridium* species. Additionally, certain *Clostridium* species are also capable of metabolizing proteins. Such protein metabolizing species that were found in high amount in the fermentor with casein are as follows: *Clostridium botulinum, Clostridium sticklandii, Clostridium tetani* and *Clostridium perfingens.* Besides their cellulose and sugar metabolizing properties these species also have extracellular enzymes metabolizing casein. With the help of the so-called Stickland reaction, characteristic to amino acid utilizing microbes, they can utilize amino acids originated from casein metabolism. In this process amino acids are used as electron donors or acceptors for the production of ATP (i.e. energy), while ammonia, CO2, and a small amount of hydrogen is produced [Ramsay IR, Pullammanappallil PC., Biodegradation.2001; 12(4):247-57. Besides protein degrading *Clostridia* species protein degrading representatives of other families were also present in the fermentor. The most abundant species in the casein containing sample are given in Table 6.

**Table 6: Composition of population in the casein containing fermentor on week 5 after initiation. 1.**

| **Species** | **Strain** | **Class** | **Order** | **Hit** |
|---|---|---|---|---|
| Slackia heliotrinireducens | Actinobacteria | Actinobacteria (class) | Coriobacteriales | 51 |
| Enterococcus faecium | Firmicutes | Bacilli | Lactobacillales | 95 |
| Staphylococcus aureus | Firmicutes | Bacilli | Bacillales | 101 |
| Bacillus pseudofirmus | Firmicutes | Bacilli | Bacillales | 120 |
| Bacillus megaterium | Firmicutes | Bacilli | Bacillales | 129 |
| Bacillus clausii | Firmicutes | Bacilli | Bacillales | 133 |
| Listeria monocytogenes | Firmicutes | Bacilli | Bacillales | 141 |
| Bacillus anthracis | Firmicutes | Bacilli | Bacillales | 146 |
| Bacillus cytotoxicus | Firmicutes | Bacilli | Bacillales | 153 |
| Enterococcus faecalis | Firmicutes | Bacilli | Lactobacillales | 154 |
| Bacillus amyloliquefaciens | Firmicutes | Bacilli | Bacillales | 154 |
| Bacillus pumilus | Firmicutes | Bacilli | Bacillales | 164 |
| Bacillus licheniformis | Firmicutes | Bacilli | Bacillales | 169 |
| Geobacillus kaustophilus | Firmicutes | Bacilli | Bacillales | 186 |
| Anoxybacillus flavithermus | Firmicutes | Bacilli | Bacillales | 198 |
| Bacillus halodurans | Firmicutes | Bacilli | Bacillales | 235 |
| Geobacillus thermodenitrificans | Firmicutes | Bacilli | Bacillales | 239 |
| Bacillus thuringiensis | Firmicutes | Bacilli | Bacillales | 310 |
| Bacillus subtilis | Firmicutes | Bacilli | Bacillales | 434 |
| Bacillus cereus | Firmicutes | Bacilli | Bacillales | 771 |
| Bacteroides thetaiotaomicron | Bacteroidetes | Bacteroidia | Bacteroidales | 135 |
| Bacteroides fragilis | Bacteroidetes | Bacteroidia | Bacteroidales | 198 |
| Parabacteroides distasonis | Bacteroidetes | Bacteroidia | Bacteroidales | 223 |
| Ralstonia solanacearum | Proteobacteria | Betaproteobacteria | Burkholderiales | 143 |
| Achromobacter piechaudii | Proteobacteria | Betaproteobacteria | Burkholderiales | 152 |
| Bordetella petrii | Proteobacteria | Betaproteobacteria | Burkholderiales | 165 |
| Bordetella avium | Proteobacteria | Betaproteobacteria | Burkholderiales | 191 |
| Bordetella pertussis | Proteobacteria | Betaproteobacteria | Burkholderiales | 202 |
| Bordetella parapertussis | Proteobacteria | Betaproteobacteria | Burkholderiales | 208 |
| Bordetella bronchiseptica | Proteobacteria | Betaproteobacteria | Burkholderiales | 218 |
| Achromobacter xylosoxidans | Proteobacteria | Betaproteobacteria | Burkholderiales | 260 |
| Thermoanaerobacter italicus | Firmicutes | Clostridia | Thermoanaerobacterales | 101 |
| Carboxydothermus hydrogenoformans | Firmicutes | Clostridia | Thermoanaerobacterales | 135 |
| Heliobacterium modesticaldum | Firmicutes | Clostridia | Clostridiales | 148 |
| Thermoanaerobacter brockii | Firmicutes | Clostridia | Thermoanaerobacterales | 151 |
| Pelotomaculum thermopropionicum | Firmicutes | Clostridia | Clostridiales | 154 |
| Clostridium sticklandii | Firmicutes | Clostridia | Clostridiales | 157 |
| Thermoanaerobacterium thermosaccharolyticum | Firmicutes | Clostridia | Thermoanaerobacterales | 158 |
| Caldicellulosiruptor saccharolyticus | Firmicutes | Clostridia | Thermoanaerobacterales | 183 |
| Clostridium cellulolyticum | Firmicutes | Clostridia | Clostridiales | 189 |
| Clostridium tetani | Firmicutes | Clostridia | Clostridiales | 249 |
| Clostridium kluyveri | Firmicutes | Clostridia | Clostridiales | 252 |
| Desulfitobacterium hafniense | Firmicutes | Clostridia | Clostridiales | 267 |
| Finegoldia magna | Firmicutes | Clostridia | Clostridiales | 283 |
| Clostridium perfringens | Firmicutes | Clostridia | Clostridiales | 367 |
| Alkaliphilus oremlandii | Firmicutes | Clostridia | Clostridiales | 410 |
| Caldanaerobacter subterraneus | Firmicutes | Clostridia | Thermoanaerobacterales | 442 |
| Clostridium thermocellum | Firmicutes | Clostridia | Clostridiales | 546 |
| Clostridium difficile | Firmicutes | Clostridia | Clostridiales | 567 |
| Alkaliphilus metalliredigens | Firmicutes | Clostridia | Clostridiales | 621 |
| Escherichia coli | Proteobacteria | Gammaproteobacteria | Enterobacteriales | 147 |
| Pseudomonas entomophila | Proteobacteria | Gammaproteobacteria | Pseudomonadales | 194 |
| Pseudomonas syringae group genomosp. 3 | Proteobacteria | Gammaproteobacteria | Pseudomonadales | 270 |
| Pseudomonas savastanoi | Proteobacteria | Gammaproteobacteria | Pseudomonadales | 283 |
| Azotobacter vinelandii | Proteobacteria | Gammaproteobacteria | Pseudomonadales | 301 |
| Pseudomonas stutzeri | Proteobacteria | Gammaproteobacteria | Pseudomonadales | 381 |
| Pseudomonas mendocina | Proteobacteria | Gammaproteobacteria | Pseudomonadales | 382 |
| Pseudomonas syringae | Proteobacteria | Gammaproteobacteria | Pseudomonadales | 625 |
| Pseudomonas putida | Proteobacteria | Gammaproteobacteria | Pseudomonadales | 652 |
| Pseudomonas aeruginosa | Proteobacteria | Gammaproteobacteria | Pseudomonadales | 774 |
| Pseudomonas fluorescens | Proteobacteria | Gammaproteobacteria | Pseudomonadales | 859 |

### Number of hits on the assembled contig

The number of species belonging to *Bacilli* family increased significantly in the fermentor. The *Bacillus cereus* is also a member of this family. With its alkaline protease this species is capable of fermenting the casein [Ramsay IR, et al: Biodegradation. 2001; 12(4):247-57]. The second most abundant species in the *Bacilli* family is the *Bacillus subtilis.* This species is capable of living both in aerobe and both in anaerobe environment: In anaerobe conditions it gets electrons from nitrates and nitrites for energy production, therefore this species plays important role in nitrogen circulation [Nakano MM, and Zuber P., Annu Rev Microbiol. 1998; 52:165-90.] It is capable of fermenting pyruvate and lactate and it obtains the protons from this process [Marino M, et al: J Bacteriol. 2001 Dec; 183(23):6815-21]. It also has proteolytic activity. The *B. subtilis* was isolated from milk and its capability of metabolizing a-lactalbumine and a-caseine was demonstrated [Feng L, et al:Proc Natl Acad Sci U S A.2007 Mar 27; 104(13):5602-7. Epub 2007 Mar 19]. The *Geobacillus thermodenitrificans* can metabolize and denitrify the long chain hydrocarbons and degrade xylanes and oligopeptides [Feng L, et al: Proc Natl Acad Sci USA. 2007 Mar 27; 104(13):5602-7. Epub 2007 Mar 19. The *Bacillus halodurans* has a number of enzymatic activities. Among its extracellular enzymes cellulases, xylanases, amylases and proteases can also be found [Feng L, et al: Proc Natl Acad Sci USA. 2007 Mar 27; 104(13):5602-7. Epub 2007 Mar 19] In anaerobe conditions the *Anoxybacillus flavithermus* which was firstly isolated from waste water ferments acetate and lactate. The *A. flavithermus* can grow even on milk powder [Saw JH, et al: Genome Biol. 2008; 9(11):R161. Epub 2008 Nov 17]. With its lipase enzyme the *Geobacillus kaustophilus* hydrolizes long chain triglycerids to diacylglycerids, monoglycerids, glycerine and fatty acids [Raja Noor Zaliha et al; Extremophiles. 2007 May; 11(3):527-35. Epub 2007 Apr 11.]. The *Bacillus licheniformis* has several extracellular enzyme activity. Its enzymes are proteases, lipases and cellulases. Sixty percent of its enzyme stock is constituted by proteolytic enzymes, thus it has significant role in fermentation of protein rich wastes [Drouin M, et al: Water Sci Technol. 2008; 57(3):423-9]. The *Bacillus pumilus* has elastinolytic and collagenolytic as well as caseinolytic activity, therefore it can utilize well the casein as substrate [Johnson BT, et al: J Med Microbiol. 2008 May; 57(Pt 5):643-51]. The *Bacillus amyloliquefaciens* is very similar to the *B. subtilis,* and this species is also capable of fermenting casein, additionally it can oxidize nitrite to nitrate. It produces organic acids from glucose, fructose, cellobiose, mannose and maltose [F. G. PRIEST, et al: INTERNATIONAL JOURNAL OF SYSTEMATIC BACTERIOLOGY, Jan. 1987, p. 69-71.]. The *Bacillus clausii* uses citrate as carbon source and capable of utilizing the hydrogen sulfide and hydrolyzing carbamide and casein [N. K. Asha Devi, et al: CURRENT SCIENCE, VOL. 95, NO. 5, 10 SEPTEMBER 2008.]. Due to its several enzymatic activities the *Bacilli* family has important role in casein containing fermentors. Species belonging to this family play important role not only in protein metabolism but also in carbon circulation, additionally they are capable of fermenting different organic acids thus enhancing the work of community adapted to casein. Due to these features the species of this family are present approximately in a similar ratio in the casein containing population as those of the members of the *Gammaproteobacteria* family. The species with proteolytic activity belonging to the *Pseudomonas* genus of the *Gammaproteobacteria* family causes that they occur in the community in such a high ratio. These species are commonly occurring in the nature, their presence in the fermentor helps the decomposition of more complicated substrates.

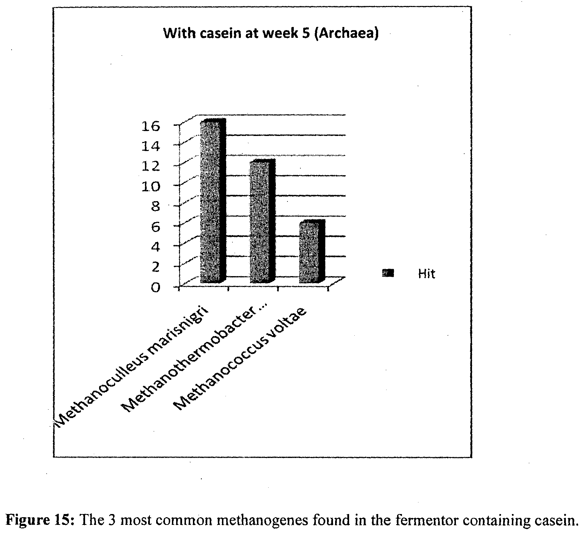

In Archaea domain of the fermentor containing casein the hidrogenotrophic *Methanoculleus marisinigri* species remained in surplus (Figure 15). The second most abundant methanogene is the hydrogen peroxide and ammonia resistant *Methanotermabacter thermoautotrophicus* species, which lives in syntrophy with the *Termanaerovibrio acidaminovorans* species, the presence of which was also detected in the casein fermentor, although in a smaller quantity than in the blood fermentor. The *Methanococcus voltae* is a hidrogenotrophic methanogene which utilizes carbon dioxide, hydrogen, and acetate for the production of methane [Whitman WB, et al: J Bacteriol. 1982 Mar; 149(3):852-63.].

### INDUSTRIAL APPLICABILITY

In the present invention the inventors confirmed that by using protein monosubstrate the biogas producing microbe community can be adapted to the decomposition of substrates having high protein content, thus to the toleration of high ammonia concentration, while the effective biogas production is maintained. Thereby the problem of co-fermentation with certain organic wastes (that results shift of the carbon/nitrogen ratio to a more favorable direction) and the accompanying disadvantages can be avoided. According to the state of art no technology is known that could accomplish the effective and stable anaerobe decomposition of protein containing monosubstrate with continuous biogas production.

The method of the invention contributes to the increase in the efficiency of biogas producing technologies at least on two fields: 1.) The applicability of the system for processing protein-rich basic materials can be characterized by determining the composition of microbe community and the relative amount of certain microbe species having diagnostic importance. Thereby the operational safety and efficiency of the biogas producing system can be improved. 2.) Having this novel information microbe formulations can be prepared as a form of clean culture or as a mixture of known bacteria strains, by the addition of which to the operating biogas fermentors the composition of the microbe community can be quickly transformed to adapt them to the changing basic material supply.

Additionally, in the use of the technology of the invention no structure modifications are necessary to carry out on the already operating biogas plants, thus at the time of establishing a biogas plant there is no need to decide whether there is an intention to decompose proteins during the years of future operations. Thus, the present technology is more cost efficient and a switch-over to it is possible any time without any particular reconstruction of the plant.

**References**

| | |
|---|---|
| Andreas Schluter ¨, Thomas Bekel, Naryttza N. Diaz, Michael Dondrup, Rudolf Eichenlaub,Karl-Heinz Gartemann, Irene Krah, Lutz Krause, Holger Kromeke ¨, The metagenome of a biogas-producing microbial community of a production-scale biogas plant fermenter analysed by the 454-pyrosequencing technology, J Biotechnol. 2008 Aug 31; 136(1-2):77-90. | 1. |
| Aragone MR, Maurizi DM, Clara LO, Navarro Estrada JL, Ascione A. Pseudomonas mendocina, an Environmental Bacterium Isolated from a Patient with Human Infective Endocarditis, J Clin Microbiol. 1992 Jun; 30(6):1583-4. | 36. |
| Bagi Z, Acs N, Bálint B, Horváth L, Dobó K, Perei KR, Rákhely G, Kovács KL. Biotechnological intensification of biogas production, Appl Microbiol Biotechnol. 2007 Aug; 76(2):473-82. | 9. |
| Bellalou J, Sakamoto H, Ladant D, Geoffroy C, Ullmann A.Deletions Affecting Hemolytic and Toxin Activities of Bordetella pertussis Adenylate Cyclase, Infect Immun. 1990 Oct; 58(10):3242-7. | 26. |
| Brambilla E, Djao OD, Daligault H, Lapidus A, Lucas S, Hammon N, Nolan M, Tice H, Cheng JF, Han C, Tapia R, Goodwin L, Pitluck S, Liolios K, Ivanova N, Mavromatis K, Complete genome sequence of Methanoplanus petrolearius type strain (SEBR 4847T), Stand Genomic Sci. 2010 Oct 27; 3(2):203-11. | 13. |
| Cadillo-Quiroz H, Yavitt JB, Zinder SH. Methanosphaerula palustris gen. nov., sp. nov., a hydrogenotrophic methanogen isolated from a minerotrophic fen peatland, Int J Syst Evol Microbiol. 2009 May; 59(Pt 5):928-35. | 15. |
| Cerdeño-Tárraga AM, Patrick S, Crossman LC, Blakely G, Abratt V, Lennard N, Poxton I, Duerden B, Harris B, Extensive DNA Inversions in the B. fragilis Genome Control Variable Gene Expression, Science. 2005 Mar 4; 307(5714):1463-5. | 21. |
| Chen, Y. et al., Inhibition of anaerobic digestion process: a review. Bioresource Technol., 99(10):4044 | |
| Chertkov O, Sikorski J, Brambilla E, Lapidus A, Copeland A, Glavina Del Rio T, Nolan M, Lucas S, Complete genome sequence of Aminobacterium colombiense type strain (ALA-1T), Stand Genomic Sci. 2010 Jun 15; 2(3):280-9. | 42. |
| Chovatia M, Sikorski J, Schröder M, Lapidus A, Nolan M, Tice H, Glavina Del Rio T, Copeland A, Cheng JF Complete genome sequence of Thermanaerovibrio acidaminovorans type strain (Su883T), Stand Genomic Sci. 2009 Nov 22; 1(3):254-61. | 43. |
| Cuetos, M.J. et al., Anaerobic digestion of solid slaughterhouse (SWW) at laboratory scale: influence of co | |
| Cuív PÓ, Klaassens ES, Durkin AS, Harkins DM, Foster L, McCorrison J, Torralba M, Nelson KE, Morrison M. Draft genome sequence of Bacteroides vulgatus PC510, a strain isolated from human faces, J Bacteriol. 2011 Aug; 193(15):4025-6. Epub 2011 May 27. | 24. |
| Demain, A.L., Newcomb, M., Wu, J.H., 2005. Cellulase, clostridia, and ethanol. Microbiol. Mol. Biol. Rev. 69, 124-154. | 10. |
| Drouin M, Lai CK, Tyagi RD, Surampalli RY. Bacillus licheniformis proteases as high value added products from fermentation of wastewater sludge: pre-treatment of sludge to increase the performance of the process, Water Sci Technol. 2008; 57(3):423-9. | 58. |
| F. G. PRIEST,1 M. GOODFELLOW, L. A. SHUTE,3 AND R. C. W. BERKELEY Bacillus amyloliquefaciens sp. nov. norn. rev, INTERNATIONAL JOURNAL OF SYSTEMATIC BACTERIOLOGY, Jan. 1987, p. 69-71. | 60. |
| Falonv G, Calmevn T, Lerov F, De Vuvst L. Coculture Fermentations of Bifidobacterium Species and Bacteroides thetaiotaomicron Reveal a Mechanistic Insight into the Prebiotic Effect of Inulin-Type Fructans, Appl Environ Microbiol. 2009 Apr; 75(8):2312-9. Epub 2009 Feb 27. | 23. |
| Feng L, Wang W, Cheng J, Ren Y, Zhao G, Gao C, Tang Y, Liu X, Han W, Peng X, Liu R, Wang L. Genome and proteome of long-chain alkane degrading Geobacillus thermodenitrificans NG80-2 isolated from a deep-subsurface oil reservoir, Proc Natl Acad Sci USA. 2007 Mar 27; 104(13):5602-7. Epub 2007 Mar 19. | 53. |
| Feng L, Wang W, Cheng J, Ren Y, Zhao G, Gao C, Tang Y, Liu X, Han W, Peng X, Liu R, Wang L. Genome and proteome of long-chain alkane degrading Geobacillus thermodenitrificans NG80-2 isolated from a deep-subsurface oil reservoir, Proc Natl Acad Sci USA. 2007 Mar 27; 104(13):5602-7. Epub 2007 Mar 19. | 54. |
| Gibb AP, Martin KM, Davidson GA, Walker B, Murphy WG. Rate of growth of Pseudomonas fluorescens in donated blood, J Clin Pathol. 1995 Aug; 48(8):717-8. | 32. |
| Gibson, A. W. and G. T. Macfarlane. Studies on the proteolytic activity of Bacteroides fragilis. J. Gen. Microbiol. Studies on the proteolytic activity of Bacteroides fragilis. J. Gen. 134: 19 | |
| Goodchild A, Raftery M, Saunders NF, Guilhaus M, Cavicchioli R. Biology of the Cold Adapted Archaeon, Methanococcoides burtonii Determined by Proteomics Using Liquid Chromatography-Tandem Mass Spectrometry, J Proteome Res. 2004 Nov-Dec; 3(6):1164-76. | 48. |
| Hejnfelt, A. and Angelidaki, I., Anaerobic digestion of slaughterhouse by | |
| Jack Maniloff, Mycoplasmas: molecular biology and pathogenesis, ASM Press, 1992. | 12. |
| Jacob-Dubuisson F, Kehoe B, Willerv E, Reveneau N, Locht C, Relman DA. Molecular characterization of Bordetella bronchiseptica filamentous haemagglutinin and its secretion machinery, Microbiology. 2000 May; 146 (Pt 5):1211-21. | 27. |
| Jae Park, Jerng Chang Yoon, Seong Joo Park, Eung Ho Kim, Yeon | |
| Johnson BT, Shaw LN, Nelson DC, Mavo JA. Extracellular proteolytic activities expressed by Bacillus pumilus isolated from endodontic and periodontal lesions, J Med Microbiol. 2008 May; 57(Pt 5):643-51. | 59. |
| Jorge Lalucat, Antoni Bennasar, Rafael Bosch, Elena García-Valdés, and Norberto J. Palleroni Biology of Pseudomonas stutzeri, Microbiol Mol Biol Rev. 2006 June; 70(2): 510-547 | 35. |
| Kato S, Kosaka T, Watanabe K, Comperative transcriptome analysis of responses of Methanothermobacter thermoautotropicus to different environmental stimuli, Environ Microbial. 2008 Apr; 10(4):893-905. Epub 2007 Nov 25. | 47. |
| Kirby AE, Metzger DJ, Murphy ER, Connell TD. Heme Utilization in Bordetella avium Is Regulated by RhuI, a Heme-Responsive Extracytoplasmic Function Sigma Factor, Infect Immun. 2001 Nov; 69(11):6951-61. | 28. |
| Krause L, Diaz NN, Edwards RA, Gartemann KH, Krömeke H, Neuweger H, Pühler A, Taxonomic composition and gene content of a methane-producing microbial community isolated from a biogas reactor, J Biotechnol. 2008 Aug 31; 136(1-2):91-101. Epub 2008 Jun 20. | 18. |
| Labutti K, Mayilraj S, Clum A, Lucas S, Glavina Del Rio T, Nolan M, Tice H, Cheng JF, Pitluck S, Liolios K, Permanent draft genome sequence of Dethiosulfovibrio peptidovorans type strain (SEBR 4207T), Stand Genomic Sci. 2010 Aug 20; 3(1):85-92 | 44. |
| Laura FRANZETTI*, Mauro SCARPELLIN. Characterisation of Pseudomonas spp. isolated from foods, Annals of Microbiology, 57 (1) 39-47 (2007) | 29. |
| Luste. S and Luostarinen, S., Anaeroboc co-digestion of meat-processing by-products and sewage sludge: effect of hygienization and organic loading rate. Bioresource Technol., 101(8):2657-2664., 2010. | |
| Marino M, Ramos HC, Hoffmann T, Glaser P, Jahn D. Modulation of Anaerobic Energy Metabolism of Bacillus subtilis by arfM (ywiD), J Bacteriol. 2001 Dec; 183(23):6815-21. | 52. |
| Maune MW, Tanner RS. Anaerobaculum hydrogeniformans sp. nov., a novel anaerobe that produces hydrogen from glucose, Int J Syst Evol Microbiol. 2011 May 20. | 41. |
| McCarthy RE, Pajeau M, Salvers AA. Role of Starch as a Substrate for Bacteroides vulgatus Growing in the Human Colon, Appl Environ Microbiol. 1988 Aug; 54(8):1911-6. | 22. |
| McInerney MJ, Brvant MP, Hespell RB, Costerton JW. Syntrophomonas wolfei gen. nov. sp. nov., an anaerobic, syntrophic, fatty acid-oxidizing bacterium, Appl Environ Microbiol. 1981 Apr; 41(4):1029-39. | 6. |
| Metcalf WW, Zhang JK, Shi X, Wolfe RS, Molecular, Genetic, and Biochemical Characterization of the serC Gene of Methanosarcina barkeri Fusaro, J Bacteriol. 1996 Oct; 178(19):5797-802. | 46. |
| N. K. Asha Devi, K. Balakrishnan, R. Gopal and S. Padmavathy Bacillus clausii MB9 from the east coast regions of India: Isolation, biochemical characterization and antimicrobial potentials, CURRENT SCIENCE, VOL. 95, NO. 5, 10 SEPTEMBER 2008. | 61. |
| Nakano MM, Zuber P. Anaerobic Growth of a 'Strict Aerobe' Bacillus subtilis, Annu Rev Microbiol. 1998; 52:165-90. | 51. |
| Nelson KE, Weinel C, Paulsen IT, Dodson RJ, Hilbert H, Martins dos Santos VA, Fouts DE, Gill SR, Pop M, Complete genome sequence and comparative analysis of the metabolically versatile Pseudomonas putida KT2440, Environ Microbiol. 2002 Dec; 4(12):799-808. | 34. |
| Nonaka H, Keresztes G, Shinoda Y, Ikenaga Y, Abe M, Naito K, Inatomi K, Furukawa K, Inui M, Yukawa H, Complete Genome Sequence of the Dehalorespiring Bacterium Desulfitobacterium hafniense Y51 and Comparison with Dehalococcoides ethenogenes 195, J Bacteriol. 2006 Mar; 188(6):2262-74. | 5. |
| Pukall R., Lapidus A., Nolan M., Copeland A. Glavina Del Rio T., Complete genome of Slackia heliotrinitreducens type strain (RHS1) Stand Genomic Sci. 2009 Nov 22; 1(3):234-41. | 2. |
| Qiu YL, Sekiguchi Y, Imachi H, Kamagata Y, Tseng IC, Cheng SS, Ohashi A, Harada H. Identification and Isolation of Anaerobic, Syntrophic Phthalate Isomer-Degrading Microbes from Methanogenic Sludges Treating Wastewater from Terephthalate Manufacturing, Appl Environ Microbiol. 2004 Mar; 70(3):1617-26. | 14. |
| Qurollo BA, Bishop PE, Hassan HM. Characterization of the iron superoxid dismutase gene of Azotobacter vinelandii: sodB may be essential for viability, Can J Microbiol. 2001 Jan; 47(1):63-71. | 38. |
| Raja Noor Zaliha Abd. Rahman; Abu Bakar Salleh; Mahiran Basri; Leow Thean Chor Lipase from Geobacillus sp. Strain T1, Extremophiles. 2007 May; 11(3):527-35. Epub 2007 Apr 11. | 57. |
| Ramsav IR, Pullammanappallil PC. Protein degradation during anaerobic wastewater treatment: derivation of stoichiometrym, Biodegradation. 2001; 12(4):247-57. | 49. |
| Ramsav IR, Pullammanappallil PC. Protein degradation during anaerobic wastewater treatment: derivation of stoichiometry, Biodegradation. 2001; 12(4):247-57. | 50. |
| Ruiz, I. et al., Treatment of slaughterhouse wastewater in UASB reactor and an anaerobic filter. Bioresource Technol., 60(3):251 | |
| Saw JH, Mountain BW, Feng L, Omelchenko MV, Hou S, Saito JA, Stott MB, Li D, Zhao G, Wu J, Galperin MY, Encapsulated in silica: genome, proteome and physiology of the thermophilic bacterium Anoxybacillus flavithermus WK1, Genome Biol. 2008; 9(11):R161. Epub 2008 Nov 17. | 56. |
| Schink B. Energetics of syntrophic cooperation in methanogenic degradation, Microbiol Mol Biol Rev. 1997 Jun; 61(2):262-80. | 7. |
| Schlüter A, Bekel T, Diaz NN, Dondrup M, Eichenlaub R, Gartemann KH, Krahn I, Krause L, Krömeke H, The metagenome of a biogas-producing microbial community of aproduction-scale biogas plant fermenter analysed by the454-pyrosequencing technology, J Biotechnol. 2008 Aug 31; 136(1-2):77-90. Epub 2008 May 27. | 17. |
| Sela DA, Chapman J, Adeuya A, Kim JH, Chen F, Whitehead TR, Lapidus A, Rokhsar DS, Lebrilla CB, German JB, Price NP, Richardson PM, Mills DA. The genome sequence of Bifidobacterium longum reflects its adaptation to the human gastrointestinal tract Proc Natl Acad Sci USA. 2008 Dec 2; 105(48):18964-9. Epub 2008 Nov 24. | 3. |
| Setubal JC, dos Santos P, Goldman BS, Ertesvåg H, Espin G, Rubio LM, Valla S, Almeida NF, Genome Sequence of Azotobacter vinelandii, an Obligate Aerobe Specialized To Support Diverse Anaerobic Metabolic Processes, J Bacteriol. 2009 Jul; 191(14):4534-45. Epub 2009 May 8. | 37. |
| Shimizu T, Ohtani K, Hirakawa H, Ohshima K, Yamashita A, Shiba T, Ogasawara N, Hattori M, Kuhara S, Havashi H. Complete genome sequence of Clostridium perfringens, an anaerobic flesh-eater, Proc Natl Acad Sci USA. 2002 Jan 22; 99(2):996-1001. Epub 2002 Jan 15. | 45. |
| Sossa, K. et al., Effect of ammonia on the methanogenic activity of methylaminotrophic methane producing Archaea enriched biofilm. Anaerobe, 10(1): 13 | |
| Stepińska MA. 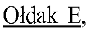 Trafny EA. **Proteolytic Activity of Pseudomonas aeruginosa Isolates with TTSS**-**Mediated Cytotoxicity and Invasiveness to Host Cells**, Curr Microbiol. 2010 May; 60(5):360-4. Epub 2009 Dec 3. | 30. |
| Takami H, Nakasone K. Takaki Y, Maeno G, Sasaki R, Masui N, Fuji F, Hirama C, Nakamura Y, Ogasawara N, Kuhara S, Horikoshi K. Complete genome sequence of the alkaliphilic bacterium Bacillus halodurans and genomic comparison with Bacillus subtilis, Nucleic Acids Res. 2000 Nov 1; 28(21):4317-31 | 55. |
| Vallet-Gely I, Novikov A Augusto L, Liehl P, Bolbach G, Péchy-Tarr M, Cosson P, Keel C. Caroff M, Lemaitre B. Hemolytic activity of Pseudomonas entomophila, a versatile soil bacterium is linked to cyclic lipopeptide production, Appl Environ Microbiol. 2010 Feb; 76(3):910-21. Epub 2009 Dec 18. | 39. |
| van Schaik W, Top J, Riley DR Boekhorst J, Vrijenhoek JE, Schapendonk CM, Hendrickx AP, Nijman IJ, Bonten MJ, Tettelin H, Willems RJ. Pyrosequencing-based comparative genome analysis of the nosocomial pathogen Enterococcus faecium and identification of a large transferable pathogenicity island, BMC Genomics. 2010 Apr 14; 11:239. | 11. |
| Vartoukian SR, Palmer RM, Wade WG. The division "Synergistes", Anaerobe. 2007 Jun-Aug; 13(3-4):99-106. Epub 2007 Jun 7. | 40. |
| Vermelho AB, Meirelles MN, Lopes A, Petinate SD, Chaia AA, Branquinha MH. Detection of Extracellular Proteases from Microorganisms on Agar Plates, Mem Inst Oswaldo Cruz. 1996 Nov-Dec; 91(6):755-60. | 31. |
| Whitman WB, Ankwanda E, Wolfe RS. Nutrition and Carbon Metabolism of Methanococculs voltae, J Bacteriol. 1982 Mar; 149(3):852-63. | 62. |
| Wu, G. et al., Effect of the solid content on anaerobic digestion of meat and bone meal. Bioresource Technol., 100(19):4326 | |
| Y Igra-Siegman, H Chmel, and C Cobbs. Clinical and laboratory characteristics of Achromobacter xylosoxidans infection. J Clin Microbiol. 1980 February; 11(2): 141-145 | 25. |
| Ye Q, Roh Y, Carroll SL, Blair B, Zhou J, Zhang CL, Fields MW. Alkaline Anaerobic Respiration: Isolation and Characterization of a Novel Alkaliphilic and Metal-Reducing Bacterium, Appl Environ Microbiol. 2004 Sep; 70(9):5595-602. | 19. |
| Ye Q, Roh Y. Carroll SL, Blair B, Zhou J, Zhang CL, Fields MW. Alkaline Anaerobic Respiration: Isolation and Characterization of a Novel Alkaliphilic and Metal-Reducing Bacterium, Appl Environ Microbiol. 2004 Sep; 70(9):5595-602. | 8. |
| Yu H, Kim BJ, Rittmann BE. Benzene, Toluene and Xylene Biodegradation by Pseudomonas putida CCMI 852, Biodegradation. 2001; 12(6):455-63. | 33. |
| Yul Roh, Chul-Min Chon and Ji-Won Moon Geosci Metal reduction and biomineralization by an alkaliphilic metal-reducing bacterium, Alkaliphilus metalliredigens (QYMF), 11(4):415-423 (2007) | 20. |
| Zhu C, Zhang J. Tang Y, Zhengkai X, Song R. Diversity of methanogenic archaea in a biogas reactor fed with swine feces as the mono-substrate by mcrA analysis, Microbiol Res. 2011 Jan 20; 166(1):27-35. Epub 2010 Jan 29. | 16. |
| Zverlov VV, Kellermann J, Schwarz WH.Functional subgenomics of Clostridium thermocellum cellulosomal genes: identification of the major catalytic components in the extracellular complex and detection of three new enzymes, Proteomics. 2005 Sep; 5(14):3646-53. | 4. |
| | |
| US6464875: Food, animal, vegetable and food preparation byproduct treatment apparatus and process | |
| GB2167639: Animal food from protein-containing waste materials | |
| JP2005161173: Method and apparatus for treating protein-containing wastewater by methane fermentation | |
| US2008000106836 A system and method for thermophilic anaerobic digester process | |
| MD2004000000005 Process sewage water biochemical treatment | |
| HU19950002140 Method for recycling partially decomposed protein waste of fibrous and woven composition and other hazardous waste by thermal disintegration and fermentation | |
| JP20050070796 Methanation method and apparatus | |
| DE19991046299 Fermentation of carbohydrate, fat and protein-containing biological waste, cellulose-containing waste, sewage sludge, paper sludge and whey | |
| SE19940001265 Production of combustible gases especially metha | |

## Claims

1. A method for the preparation of a microorganism consortium, said consortium being capable of fermenting, together with a simultaneous production of methane-containing biogas, a substrate having at least 75% protein content, preferably at least 90% protein content and an organic dry-matter content of at least 4 g/l, preferably 5 g/l, more preferably at least 6 g/l relative to the fermentor volume, by adaptation to said substrate, said method comprising the steps of
- providing a biogas-producing microorganism consortium cultured until complete fermentation preferably on animal manure or plant substrate or on a mixture thereof,
- adding the microorganism consortium and a protein monosubstrate to a biogas fermentor,
- culturing the microorganism consortium on the protein monosubstrate thereby inducing the fermentation of the protein containing substrate,
- adding further portions of the protein monosubstrate multiple times to the fermentation mixture in the biogas fermentor during culturing time,
- monitoring the fermentation process in the fermentation mixture,
- determining that the consortium is stable i.e. when at least during the fermentation the concentration of one or more measured organic acids remains essentially unchanged, i.e. the change in concentration of the measured organic acids is not more than 30%, and the change in biogas yield is not more than 30% for at least approximately 3 weeks,
and is adapted to the substrate once the consortium is found stable upon monitoring, and preferably determining that the consortium produces biogas of at least 50% methane-content on the substrate,
- collecting the microorganism consortium which is stable and adapted to the substrate from the fermentation mixture,
wherein the biogas producing microorganism consortium comprises at least proteolytic and/or peptidolytic, acetogenic and methanogenic microorganisms.

2. The method according to claim 1 wherein the protein-containing substrate is added to the fermentation mixture at given time intervals, preferably at regular time intervals, preferably during a first time-period in equal portions and then during a second time-period in increasing portions.

3. A method according to claim 1 or 2 wherein upon monitoring the fermentation process one or more step(s) is/are carried out from the following steps on at least two occasions:
- obtaining data on the composition of the microorganism-consortium in the fermentation broth, preferably by a metagenomic method,
- determining the concentration of one or more volatile organic acid (preferably acetic acid and/or propionic acid) in the biogas fermentor, preferably at most 1 or 2 days before addition of the substrate portion,
- determining the protease activity in the fermentation mixture, preferably at most 120 minutes, preferably at most 90 minutes, highly preferably at most 60 minutes, 40 minutes or 30 minutes after the addition of the substrate portion,
- measuring the amount of the biogas obtained in the biogas fermentor.

4. A method according to claim 3 wherein
- data are obtained on the composition of the microorganism consortium in the fermentation mixture by a metagenomic method from sample(s) taken at least on two occasions, and if at least on the second occasion, as compared to the data obtained for the first time, the microorganism consortium
- comprises bacterium species of the Gammaproteobacteria class, preferably of the Pseudomonadales order in a significantly increased ratio, preferably in at least 20%, more preferably in 20 to 50%, highly preferably in 22-40%, and
comprises bacterium species of the Clostridia class, preferably of the Clostridiales order and the Thermoanaerobacterales order in a significantly reduced ratio, preferably in at most 60%, more preferably in 10 to 40%, highly preferably in 20-30%,
this finding is considered as data indicative of adaptation.

5. The method according to claim 4, wherein
the adapted microorganism consortium among the species of the Clostridiales order comprises
- a species of cellulolytic activity
- a species being capable of reducing metal ions, preferably iron, cobalt, manganese and selenium, and/or among the species of the Pseudomonadales order comprises
- species of proteolytic activity.

6. A method according to claim 3, wherein
i) at least 3, 4, 5, 6, 7 or 8 times
- the concentration of one or more volatile organic acid is determined in the biogas fermentor and/or
- the protease activity is determined in the fermentation mixture, and preferably
- the quantity of biogas produced in the biogas fermentor is measured in comparison with the protein content of the added substrate and
ii)
- based on these data the amount of substrate added is determined and/or
- based on these data so obtained stability of the microorganism consortium and/or adaptation of the microorganism consortium to the substrate is characterized.

7. A biogas producing microorganism consortium which comprises proteolytic and/or peptidolytic, acetogenic and methanogenic microorganisms and which is obtainable according to any of claims 1 to 6, wherein
said biogas producing microorganism consortium is capable of fermenting a substrate having a protein content of at least 75%, preferably at least 90% and an organic dry-matter content of at least 4 g/l, preferably 5 g/l, more preferably at least 6 g/l relative to the fermentor volume, with a concomitant production of methane-containing biogas, and comprises
- proteolytic bacteria of the Gammaproteobacteria class,
- denitrifying bacteria,
- cellulolytic, peptide degrading and/or amino acid degrading and/or thiosulphate reducing bacteria of the Clostridia class, and preferably
- the consortium comprises at least 20%, preferably 20 to 50%, more preferably 22 to 40 % bacteria belonging to the Gammaproteobacteria class, preferably to the Pseudomonadales order, and
- the consortium comprises less than 50%, more preferably 10 to 40%, highly preferably 20 to 30% bacteria belonging to species of the Clostridia class, preferably of the Clostridiales order and of the Thermoanaerobacterales order,
wherein the biogas producing microorganism consortium is capable of fermenting a protein monosubstrate and preferably is capable of producing biogas of at least 500 ml/g, preferably at least 600 ml/g, highly preferably at least 700 ml/g in relation to organic dry-matter content, and is capable of maintaining the production of biogas for at least 1 month or more preferably for at least 2 months.

8. A microorganism consortium of claim 7 further comprising
- a metal-ion reducing bacterium of the Clostridia class, and/or
- a methanogenic bacterium of the Clostridia class, and/or
- an amino acid degrading and/or sugar degrading and/or thiosulphate reducing bacterium of the Synergistia class and/or
- a proteolytic and/or nitrate fermenting and/or nitrite fermenting bacterium of the Bacilli class,
- a methanogenic microorganism of the Archea domain,
wherein preferably
- the proteolytic bacterium of the Gammaproteobacteria class belongs to the Pseudomonadales order, and preferably is selected from at least Pseudomonas aeruginosa, Pseudomonas entomophila, Pseudomonas fluorescens and Pseudomonas putida,
- the denitrifying bacterium is a bacterium of the Gammaproteobacteria class and/or of the Synergistia class, preferably a bacterium belonging to the Pseudomonadales order and/or to the Synergistales order, highly preferably is selected from at least Pseudomonas stutzeri and Azobacter vinelandii,
- the cellulolytic, peptide degrading and/or amino acid degrading bacterium of the Clostridia class preferably belongs to Clostridiales order, preferably is selected from at least Clostridium celloliticum, Clostridium sticklandii, Clostridium perfingens, Alkaliphilus peptidofermentans and Desulfitobacterium hafniense.

9. The microorganism consortium of claim 8 wherein
- the metal ion reducing bacterium of the Clostridia class is preferably selected from Alkaliphilus metalliredigens and Desufotomaculum reducens and/or
- the hydrogen producing and polymer degrading bacterium of the Clostridia class belongs to the Thermoanaerobacterales order and is preferably Caldicellulosiruptor saccharoliticus, and/or
- the amino acid degrading and/or sugar degrading and/or thisulphate degrading bacterium of the Synergistia class belongs to the Synergistales order, and is preferably selected from Aminobacter colombiense, Anaerobaculum hydrogeniformans, Termoanaerovibrio acidaminovorans and Dethiosulfovibrio-peptidovorans, and/or
- the proteolytic and/or nitrate fermenting and/or nitrite fermenting bacterium of the Bacilli class belongs to the Bacillales order and is preferably selected from at least Bacilllus cereus, Bacillus amyloliquefaciens and Bacillus subtilis,
- the methanogenic microorganism of the Archea domain is selected from a microorganism of the Methanosarcina, Methanothermobacter, Methanococcoides, Methanoculleus and Methanococcus genera and is preferably selected from Methanosarcina barkeri, Methanothermobacter thermoautotrophicus, Methanococcoides burtonii, Methanoculleus marisnigri and Methanococcus voltae, highy preferably is a Methanothermobacter species living in syntropy with Termoanaerovibrio acidaminovorans, highly preferably Methanothermobacter thermoautotrophicus, or
- Pseudomonas fluorescens.

10. Use of a microorganism consortium according to any of claims 7 to 9 for fermenting a substrate of high protein content with a concomitant production of methane containing biogas.

11. Use of a microorganism consortium according to any of claims 7 to 9 for increasing biogas production by addition of the microorganism consortium to a biogas fermentor comprising a substrate of protein content or by addition of a protein containing substrate to a biogas fermentor comprising the microorganism consortium.

12. A method for fermenting a substrate of high protein content with a production of biogas, **characterized in that**
- in the biogas fermentor a fermentation mixture comprising a microorganism consortium according to any of claims 7 to 9 and a substrate of at least 70% protein content is provided,
- the fermentation mixture is fermented,
- the biogas so produced is collected and/or utilized.

13. The method for fermenting a substrate of high protein content with a production of biogas according to claim 12 **characterized in that**
- upon fermentation protein monosubstrate portions are added to the fermentation mixture into the biogas fermentor during the time of culturing.

14. The method for fermenting a substrate of high protein content with a production of biogas according to claim 13, wherein fermentation is maintained for at least 1 month, preferably for at least 2 months, and
- preferably the biogas is continuously collected or utilized during fermentation.

## Patentansprüche

1. Verfahren zur Herstellung eines Mikroorganismuskonsortiums, wobei das genannte Konsortium, zusammen mit gleichzeitiger Produktion von Biogas mit Methangehalt, zur Fermentation eines Substrates mit mindestens 75% Proteingehalt, vorzugsweise mindestens 90% Proteingehalt und, bezogen auf das Fermentorvolumen, mit mindestens 4 g/l, vorzugsweise 5 g/l, bevorzugt mindestens 6 g/l Feststoffgehalt durch Anpassung an das genannte Substrat, welches Verfahren die folgenden Schritte enthält:
- Bereitstellung eines Mikroorganismuskonsortiums zur Produktion von Biogas, welches bis zur vollständigen Fermentation, vorzugsweise an Tierdünger oder Pflanzensubstrat oder einer Mischung von diesen, kultiviert ist,
- Zugabe des Mikroorganismuskonsortiums und eines Proteinmonosubstrates zu einem Biogasfermentor,
- Kultivierung des Mikroorganismuskonsortiums an dem Proteinmonosubstrat und dadurch Indizierung der Fermentation des Substrates mit Proteingehalt,
- mehrmalige Zugabe weiterer Anteile von Proteinmonosubstrat zu dem Fermentationsgemisch in dem Biogasfermentor während des Zeitraums der Kultivierung,
- Überwachung des Verlaufs der Fermentation in dem Fermentationsgemisch,
- Bestimmung, dass das Konsortium stabil ist, d.h. wenn mindestens während der Fermentation die Konzentration einer oder mehrerer gemessener organischer Säuren wesentlich unverädert bleibt, d.h. die Veränderung der Konzentration der gemessenen organischen Säuren beträgt nicht mehr als 30% und die Veränderung der Ausbeute von Biogas beträgt nicht mehr als 30% für mindestens etwa drei Wochen,
und ist an das Substrat angepasst, wenn das Konsortium durch die Überwachung für stabil befunden wurde, und vorzugsweise Bestimmung, dass das Konsortium an dem Substrat ein Biogas mit mindestens 50% von Methangehalt produziert,
- Auffangen des Mikroorganismuskonsortiums von dem Fermentationsgemisch, welches stabil und an das Substrat angepasst ist,
wobei das biogasproduzierende Mikroorganismuskonsortium mindestens proteolytische und/oder peptidolytische, acetogene und methanogene Mikroorganismen enthält.

2. Verfahren nach Anspruch 1, wobei das Substrat mit Proteingehalt in bestimmten Zeitabständen, vorzugsweise in gleichmäßigen Zeitabständen, vorzugsweise in einer ersten Zeitperiode in gleichen Anteilen und dann in einer zweiten Zeitperiode in ansteigenden Anteilen zugegeben wird.

3. Verfahren nach Anspruch 1 oder 2, wobei während der Überwachung des Verlaufs der Fermentation ein oder mehrere Schritte gewählt aus den folgenden Schritten mindestens zweimal durchgeführt werden:
- Erhalten von Angaben über die Zusammensetzung des Mikroorganismuskonsortiums in dem Fermentationsgemisch, vorzugsweise durch eine metagenomische Methode,
- Bestimmung der Konzentration einer oder mehrerer flüchtiger organischer Säuren, vorzugsweise Essigsäure und/oder Propionsäure, in dem Biogasfermentor, vorzugsweise höchstens 1 oder 2 Tage vor der Zugabe von dem Substratanteil,
- Bestimmung der Proteaseaktivität in dem Fermentationsgemisch, vorzugsweise höchstens 120 Minuten, vorzugsweise höchstens 90 Minuten, insbesondere höchstens 60 Minuten, 40 Minuten oder 30 Minuten nach der Zugabe von dem Substratanteil,
- Messung der Menge des in dem Biogasfermentor gewonnenen Biogases.

4. Verfahren nach Anspruch 3, wobei
- Angaben über die Zusammensetzung des Mikroorganismuskonsortiums in dem Fermentationsgemisch durch eine metagenomische Methode von Probe(n), welche mindestens zu zwei Gelegenheiten genommen wurden, erhalten werden, und wenn mindestens bei der zweiten Gelegenheit, im Vergleich mit den Angaben von der ersten Gelegenheit, das Mikroorganismuskonsortium
- Bakterienarten der Klasse Gammaproteobacteria, vorzugsweise der Ordnung Pseudomonadales in wesentlich erhöhtem Anteil, vorzugsweise mindestens um 20%, bevorzugt um 20 bis 50%, höchstens bevorzugt um 22-40% enthält, und
- Bakterienarten der Klasse Clostridia, vorzugsweise der Ordnung Clostridiales und der Ordnung Thermoanaerobacterales in wesentlich reduziertem Anteil, vorzugsweise um höchstens 60%, bevorzugt um 10 bis 40%, höchstens bevorzugt um 20-30% enthält,
dieses Ergebnis als die Anpassung bezeichnende Angabe berücksichtigt wird.

5. Verfahren nach Anspruch 4, wobei
das angepasste Mikroorganismuskonsortium unter den Arten der Ordnung Clostridiales
- eine Art mit cellulolytischer Aktivität
- eine zur Reduzierung von Metallionen, vorzugsweise Eisen, Cobalt, Mangan und Selen, geeigneten Art, und/oder unter den Arten der Ordnung Pseudomonadales
- Arten mit proteolytischer Aktivität enthält.

6. Verfahren nach Anspruch 3, wobei
i) mindestens 3, 4, 5, 6, 7 oder 8-mal
- die Konzentration einer oder mehrerer flüchtiger organischer Säuren in dem Biogasfermentor bestimmt wird und/oder
- die Proteaseaktivität des Fermentationsgemisches bestimmt wird, und vorzugsweise
- die Menge des in dem Biogasfermentor erhaltenen Biogases im Bezug auf den Proteingehalt des zugegebenen Substrates gemessen wird und
ii)
- die Menge an dem zugegebenen Substrat aufgrund dieser Angaben bestimmt wird und/oder
- die Stabilität des Mikroorganismuskonsortiums und/oder die Anpassung des Mikroorganismuskonsortiums aufgrund der so gewonnenen Angaben gekennzeichnet wird.

7. Ein Mikroorganismuskonsortium zur Produktion von Biogas, welches proteolytische und/oder peptidolytische, acetogene und methanogene Mikroorganismusen enthält und welches nach einem der Ansprüche 1 bis 6 herstellbar ist, wobei
das genannte Mikroorganismuskonsortium zur Produktion von Biogas, zur Fermentation eines Substrates, bezogen auf das Fermentorvolumen, mit einem Proteingehalt von mindestens 75%, vorzugsweise mindestens 90%, und mit einem organischen Feststoffgehalt von mindestens 4 g/l, vorzugsweise 5 g/l, bevorzugt mindestens 6 g/l, mit gleichzeitiger Produktion von Biogas mit Methangehalt geeignet ist, und
- proteolytisches Bakterium der Klasse Gammaproteobacteria,
- denitrifizierendes Bakterium,
- cellulolytisches, peptiddegradierendes und/oder aminosäuredegradierendes und/oder thiosulphatreduzierendes Bakterium der Klasse Clostridia enthält, und vorzugsweise
- das Konsortium mindestens 20%, vorzugsweise 20 bis 50%, bevorzugt 22 bis 40 % Bakterium, welches zur Klasse Gammaproteobacteria, vorzugsweise zur Ordnung Pseudomonadales gehört, enthält, und
- das Konsortium höchstens 50%, bevorzugt 10 bis 40%, insbesondere 20 bis 30% Bakterium, welches zu Arten der Klasse Clostridia, vorzugsweise der Ordnung Clostridiales und der Ordnung Thermoanaerobacterales gehört, enthält,
wobei das Mikroorganismuskonsortium zur Produktion von Biogas, zur Fermentation eines Proteinmonosubstrates geeignet ist und vorzugsweise zur Produktion von Biogas von mindestens 500 ml/g, vorzugsweise mindestens 600 ml/g, insbesondere mindestens 700 ml/g in Relation zum organischen Feststoffgehalt, geeignet ist und zur Aufrechterhaltung der Produktion von Biogas für mindestens 1 Monat oder bevorzugt für mindestens 2 Monate geeignet ist.

8. Das Mikroroganismuskonsortium nach Anspruch 7, welches weiterhin
- ein metallionreduzierendes Bakterium der Klasse Clostridia, und/oder
- ein methanogenes Bakterium der Klasse Clostridia, und/oder
- ein aminosäuredegradierendes und/oder zuckerdegradierendes und/oder thiosulphatreduzierendes Bakterium der Klasse Synergistia und/oder
- ein proteolytisches und/oder nitratfermentierendes und/oder nitritfermentierendes Bakterium der Klasse Bacilli,
- einen methanogenen Mikroorganismus der Domäne Archaea, enthält,
wobei vorzugsweise
- das proteolytische Bakterium der Klasse Gammaproteobacteria zur Ordnung Pseudomonadales gehört, und vorzugsweise mindestens aus Pseudomonas aeruginosa, Pseudomonas entomophila, Pseudomonas fluorescens und Pseudomonas putida gewählt ist,
- das denitrifizierende Bakterium ein Bakterium der Klasse Gammaproteobacteria und/oder der Klasse Synergistia ist, vorzugsweise ein Bakterium, welches zur Ordnung Pseudomonadales und/oder zur Ordnung Synergistales gehört, insbesondere mindestens aus Pseudomonas stutzeri und Azobacter vinelandii gewählt ist,
- das cellulolytische, peptiddegradierende und/oder aminosäuredegradierende Bakterium der Klasse Clostridia vorzugsweise zur Ordnung Clostridiales gehört, vorzugsweise mindestens aus Clostridium celloliticum, Clostridium sticklandii, Clostridium perfingens, Alkaliphilus peptidofermentans und Desulfitobacterium hafniense gewählt ist.

9. Das Mikroorganismuskonsortium nach Anspruch 8, wobei
- das metallionreduzierende Bakterium der Klasse Clostridia vorzugsweise aus Alkaliphilus metalliredigens und Desufotomaculum reducens gewählt ist, und/oder
- das wasserstoffproduzierende und polymerdegradierende Bakterium der Klasse Clostridia zur Ordnung Thermoanaerobacterales gehört und vorzugsweise Caldicellulosiruptor saccharoliticus ist, und/oder
- das aminosäuredegradierende und/oder zuckerdegradierende und/oder thiosulphatdegradierende Bakterium der Klasse Synergistia zur Ordnung Synergistales gehört, und vorzugsweise aus Aminobacter colombiense, Anaerobaculum hydrogeniformans, Termoanaerovibrio acidaminovorans und Dethiosulfovibrio peptidovorans gewählt ist, und/oder
- das proteolytische und/oder nitratfermentierende und/oder nitritfermentierende Bakterium der Klasse Bacilli zur Ordnung Bacillales gehört, und vorzugsweise mindestens aus Bacillus cereus, Bacillus amyloliquefaciens and Bacillus subtilis gewählt ist,
- der methanogene Mikroorganismus der Domäne Archaea aus einem Mikroorganismus der Gattung Methanosarcina, Methanothermobacter, Methanococcoides, Methanoculleus und Methanococcus und vorzugsweise aus Methanosarcina barkeri, Methanothermobacter thermoautotrophicus, Methanococcoides burtonii, Methanoculleus marisnigri und Methanococcus voltae gewählt ist, insbesondere eine Art Methanothermobacter ist, welche in Syntropie mit Termoanaerovibrio acidaminovorans lebt, insbesondere Methanothermobacter thermoautotrophicus ist, oder
- Pseudomonas fluorescens ist.

10. Verwendung eines Mikroorganismuskonsortiums nach einem der Ansprüche 7 bis 9 zur Fermentation eines Substrates mit hohem Proteingehalt mit gleichzeitiger Produktion von Biogas mit Methangehalt.

11. Verwendung eines Mikroorganismuskonsortiums nach einem der Ansprüche 7 bis 9 zur Erhöhung der Biogasproduktion durch Zugabe des Mikroorganismuskonsortiums zu einem Biogasfermentor, welcher ein Substrat mit Proteingehalt enthält, oder durch Zugabe eines Substrates mit Proteingehalt zu einem Biogasfermentor, welcher das Mikroorganismuskonsortium enthält.

12. Verfahren zur Fermentierung eines Substrates mit hohem Proteingehalt mit der Produktion von Biogas, **dadurch gekennzeichnet, dass**
- in dem Biogasfermentor ein Fermentationsgemisch, welches ein Mikroorganismuskonsortium nach einem der Ansprüche 7 bis 9 und ein Substrat mit mindestens 70% Proteingehalt enthält, bereitgestellt ist,
- das Fermentationsgemisch fermentiert ist,
- das gewonnene Biogas aufgefangen und/oder verwendet ist.

13. Das Verfahren zur Fermentierung eines Substrates mit hohem Proteingehalt mit der Produktion von Biogas nach Anspruch 12, **dadurch gekennzeichnet, dass**
- während der Fermentation Anteile vom Proteinmonosubstrat zu dem Fermentationsgemisch in dem Biogasfermentor während der Zeitperiode der Kultivierung zugegeben werden.

14. Das Verfahren zur Fermentierung eines Substrates mit hohem Proteingehalt mit der Produktion von Biogas nach Anspruch 13, wobei die Fermentation mindestens für 1 Monat, vorzugsweise mindestens 2 Monate aufrechterhalten ist, und
- vorzugsweise das Biogas unter der Fermentation durchgehend aufgefangen oder verwendet wird.

## Revendications

1. Procédé de préparation d'un consortium de microorganismes, ledit consortium étant capable de fermenter, conjointement avec une production simultanée de biogaz contenant du méthane, un substrat ayant au moins 75% de teneur en protéines, de préférence au moins 90% de teneur en protéines et une teneur en matière sèche organique d'au moins 4 g/l, de préférence 5 g/l, plus préférablement au moins 6 g/l par rapport au volume fermenteur, par adaptation audit substrat, ledit procédé comprenant les étapes de
- fournir un consortium de microorganismes producteur de biogaz cultivé jusqu'à la fermentation complète de préférence sur du fumier animal ou du substrat végétal ou sur un mélange de ceux-ci,
- ajouter le consortium de microorganismes et un monosubstrat de protéine à un fermenteur de biogaz,
- cultiver le consortium de microorganismes sur le monosubstrat de protéine induisant ainsi la fermentation du substrat contenant la protéine,
- ajouter des portions supplémentaires du monosubstrat de protéine plusieurs fois au mélange de fermentation dans le fermenteur de biogaz pendant le temps de culture,
- contrôler le processus de fermentation dans le mélange de fermentation,
- déterminer que le consortium est stable *i.e.* lorsqu'au moins pendant la fermentation la concentration d'un ou plusieurs acides organiques mesurés reste essentiellement inchangée, *i.e.* le changement en concentration des acides organiques mesurés n'est pas plus de 30%, et le changement en rendement de biogaz n'est pas plus de 30% pour au moins approximativement 3 semaines,
et est adapté au substrat une fois que le consortium est trouvé stable lors du contrôle, et de préférence déterminer que le consortium produit du biogaz d'au moins 50% de teneur en méthane sur le substrat,
- prélever le consortium de microorganismes qui est stable et adapté au substrat du mélange de fermentation,
dans lequel le consortium de microorganismes produisant du biogaz comprend au moins des microorganismes protéolytiques et/ou peptidolytiques, acétogéniques et méthanogéniques.

2. Procédé selon la revendication 1 dans lequel le substrat contenant la protéine est ajouté au mélange de fermentation à des intervalles de temps donnés, de préférence à des intervalles de temps réguliers, de préférence pendant une première période de temps en portions égales et ensuite pendant une deuxième période de temps en portions croissantes.

3. Procédé selon la revendication 1 ou 2 dans lequel lors du contrôle du processus de fermentation une ou plusieurs étape(s) est/sont exécutée(s) à partir des étapes suivantes à au moins à deux occasions :
- obtenir des données sur la composition du consortium de microorganismes dans le bouillon de fermentation, de préférence par un procédé de métagénomique,
- déterminer la concentration d'un ou plusieurs acides organiques volatils (de préférence l'acide acétique et/ou l'acide propionique) dans le fermenteur de biogaz, de préférence au plus 1 ou 2 jours avant l'addition de la portion de substrat,
- déterminer l'activité de la protéase dans le mélange de fermentation, de préférence au plus 120 minutes, de préférence au plus 90 minutes, de manière hautement préférée au plus 60 minutes, 40 minutes ou 30 minutes après l'addition de la portion de substrat,
- mesurer la quantité de biogaz obtenue dans le fermenteur de biogaz.

4. Procédé selon la revendication 3 dans lequel
- des données sont obtenues sur la composition du consortium de microorganismes dans le mélange de fermentation par un procédé de métagénomique à partir d'échantillon(s) prélevé(s) au moins à deux occasions, et si au moins à la deuxième occasion, tel que comparé aux données obtenues pour la première fois, le consortium de microorganismes
- comprend des espèces bactériennes de la classe des *Gammaprotéobactéries,* de préférence de l'ordre des *Pseudomonadales* dans un rapport significativement augmenté, de préférence dans au moins 20%, plus préférablement dans 20 à 50%, de manière hautement préférée dans 22-40%, et
comprend des espèces bactériennes de la classe des *Clostridia,* de préférence de l'ordre des *Clostridiales* et de l'ordre des *Thermoanaerobacterales* dans un rapport significativement réduit, de préférence dans au plus 60%, plus préférablement dans 10 à 40%, de manière hautement préférée dans 20-30%,
cette découverte est considérée comme des données indicatives d'adaptation.

5. Procédé selon la revendication 4, dans lequel
le consortium de microorganismes adapté parmi les espèces de l'ordre des *Clostridiales* comprend
- une espèce d'activité cellulolytique
- une espèce étant capable de réduire les ions métalliques, de préférence le fer, le cobalt, le manganèse et le sélénium, et/ou parmi les espèces de l'ordre des *Pseudomonadales* comprend
- des espèces d'activité protéolytique.

6. Procédé selon la revendication 3, dans lequel
i) au moins 3, 4, 5, 6, 7 ou 8 fois
- la concentration d'un ou plusieurs acides organiques volatils est déterminée dans le fermenteur de biogaz et/ou
- l'activité de la protéase est déterminée dans le mélange de fermentation, et de préférence
- la quantité de biogaz produite dans le fermenteur de biogaz est mesurée par comparaison avec la teneur en protéine du substrat ajouté et
ii)
- sur la base de ces données la quantité de substrat ajouté est déterminée et/ou
- sur la base de ces données la stabilité du consortium de microorganismes ainsi obtenue et/ou l'adaptation du consortium de microorganismes au substrat est caractérisée.

7. Consortium de microorganismes producteur de biogaz qui comprend des microorganismes protéolytiques et/ou peptidolytiques, acétogènes et méthanogénes et qui peut être obtenu selon l'une quelconque des revendications 1 à 6, dans lequel
ledit consortium de microorganismes producteurs de biogaz est capable de fermenter un substrat ayant une teneur en protéines d'au moins 75%, de préférence au moins 90% et une teneur en matière sèche organique d'au moins 4 g/l, de préférence 5 g/l, plus préférablement au moins 6 g/l par rapport au volume fermenteur, avec une production concomitante de biogaz contenant du méthane, et comprend
- des bactéries protéolytiques de la classe des *Gammaproteobacteria,*
- des bactéries dénitrifiantes,
- des bactéries cellulolytiques, dégradant le peptide et/ou dégradant les acides aminés et/ou réduisant le thiosulfate de la classe des *Clostridia,* et de préférence
- le consortium comprend au moins 20%, de préférence 20 à 50%, plus préférablement 22 à 40% de bactéries appartenant à la classe des *Gammaproteobacteria,* de préférence à l'ordre des *Pseudomonadales,* et
- le consortium comprend moins de 50%, plus préférablement 10 à 40%, de manière hautement préférée 20 à 30% de bactéries appartenant à des espèces de la classe des *Clostridia,* de préférence de l'ordre des *Clostridiales* et de l'ordre des *Thermoanaerobacterales,*
dans lequel le consortium de microorganismes producteurs de biogaz est capable de fermenter un monosubstrat de protéine et de préférence est capable de produire du biogaz d'au moins 500 ml/g, de préférence au moins 600 ml/g, de manière hautement préférée au moins 700 ml/g en relation avec la teneur en matière sèche organique, et est capable de maintenir la production de biogaz pendant au moins 1 mois ou plus préférablement pendant au moins 2 mois.

8. Consortium de microorganismes selon la revendication 7 comprenant en outre
- une bactérie réduisant les ions métalliques de la classe des *Clostridia,* et/ou
- une bactérie méthanogène de la classe des *Clostridia,* et/ou
- une bactérie dégradant les acides aminés et/ou dégradant le sucre et/ou réduisant le thiosulfate de la classe des *Synergistia* et/ou
- une bactérie protéolytique et/ou fermentant le nitrate et/ou fermentant le nitrite de la classe des *Bacilli,*
- un microorganisme méthanogène du domaine des *Archea,*
dans lequel de préférence
- la bactérie protéolytique de la classe des *Gammaprotéobactéries* appartient à l'ordre des *Pseudomonadales,* et est de préférence sélectionnée parmi au moins *Pseudomonas aeruginosa, Pseudomonas entomophila, Pseudomonas fluorescens* et *Pseudomonas putida,*
- la bactérie dénitrifiante est une bactérie de la classe des *Gammaprotéobactéries* et/ou de la classe des *Synergistia,* de préférence une bactérie appartenant à l'ordre des *Pseudomonadales* et/ou à l'ordre des *Synergistales,* de manière hautement préférée est sélectionnée parmi au moins *Pseudomonas stutzeri* et *Azobacter vinelandii,*
- la bactérie cellulolytique, dégradant le peptide et/ou dégradant les acides aminés de la classe des *Clostridia* appartient de préférence à l'ordre des *Clostridiales,* est de préférence sélectionnée parmi au moins *Clostridium celloliticum, Clostridium sticklandii, Clostridium perfingens, Alkaliphilus peptidofermentans* et *Desulfitobacterium hafniense.*

9. Consortium de microorganismes selon la revendication 8 dans lequel
- la bactérie réduisant les ions métalliques de la classe des *Clostridia* est de préférence sélectionnée parmi *Alkaliphilus metalliredigens* et *Desufotomaculum reducens* et/ou
- la bactérie produisant de l'hydrogène et dégradant le polymère de la classe des *Clostridia* appartient à l'ordre des *Thermoanaerobacterales* et est de préférence *Caldicellulosiruptor saccharoliticus,* et/ou
- la bactérie dégradant les acides aminés et/ou dégradant le sucre et/ou dégradant le thiosulfate de la classe des *Synergistia* appartient à l'ordre des *Synergistales,* et est de préférence sélectionnée parmi *Aminobacter colombiense, Anaerobaculum hydrogeniformans, Termoanaerovibrio acidaminovorans* et *Dethiosulfovibrio-peptidovorans,* et/ou
- la bactérie protéolytique et/ou fermentant le nitrate et/ou fermentant le nitrite de la classe des *Bacilli* appartient à l'ordre des *Bacillales* et est de préférence sélectionnée parmi au moins *Bacilus cereus, Bacillus amyloliquefaciens et Bacillus subtilis,*
- le microorganisme méthanogène du domaine des *Archea* est sélectionné parmi un microorganisme du genre *Methanosarcina, Methanothermobacter, Methanococcoides, Methanoculleus* et *Methanococcus* et est de préférence sélectionée parmi *Methanosarcina barkeri, Methanothermobacter thermoautotrophicus, Methanococcoides burtonii, Methanoculleus marisnigri* et *Methanococcus voltae,* de manière hautement préférée est une espèce *Methanothermobacter* vivant en syntropie avec *Termoanaerovibrio acidaminovorans,* de manière hautement préférée *Methanothermobacter thermoautotrophicus,* ou
- *Pseudomonas fluorescens.*

10. Utilisation d'un consortium de microorganismes selon l'une quelconque des revendications 7 à 9 pour fermenter un substrat de haute teneur en protéine avec une production concomitante de biogaz contenant du méthane.

11. Utilisation d'un consortium de microorganismes selon l'une quelconque des revendications 7 à 9 pour augmenter la production de biogaz par addition du consortium de microorganismes à un fermenteur de biogaz comprenant un substrat de teneur en protéine ou par addition d'un substrat contenant une protéine à un fermenteur de biogaz comprenant le consortium de microorganismes.

12. Procédé de fermentation d'un substrat à haute teneur en protéine avec production de biogaz, **caractérisé en ce que**
- dans le fermenteur de biogaz, un mélange de fermentation comprenant un consortium de microorganismes selon l'une quelconque des revendications 7 à 9 et un substrat d'au moins 70% de teneur en protéine est pourvu,
- le mélange de fermentation est fermenté,
- le biogaz ainsi produit est collecté et/ou utilisé.

13. Procédé de fermentation d'un substrat à haute teneur en protéine avec production de biogaz selon la revendication 12 **caractérisé en ce que**
- lors de la fermentation des portions de monosubstrat de protéine sont ajoutées au mélange de fermentation dans le fermenteur de biogaz pendant le temps de culture.

14. Procédé de fermentation d'un substrat à haute teneur en protéine avec production de biogaz selon la revendication 13, dans lequel la fermentation est maintenue pendant au moins 1 moins, de préférence pendant au moins 2 mois, et
- de préférence le biogaz est collecté ou utilisé en continu pendant la fermentation.
